# EUROPÄISCHE PATENTSCHRIFT

(11) **EP 2 847 334 B1**
(45) Veröffentlichungstag und Bekanntmachung des Hinweises auf die Patenterteilung: **18.12.2019**
(21) Anmeldenummer: 13723041.3
(22) Anmeldetag: 02.05.2013
(51) Int. Cl.: C12N 15/29, C12N 5/10, C12N 15/81, C12N 15/62, C12N 11/00, C07K 14/415

(54) **VERWENDUNG ARTIFIZIELLER FORISOMENKÖRPER ZUR ENZYMIMMOBILISIERUNG ODER ZUR AUFREINIGUNG REKOMBINANTER PROTEINE**
USE OF ARTIFICIAL FORISOME BODIES FOR ENZYME IMMOBILISATION OR PURIFICATION OF RECOMBINANT PROTEINS
UTILISATION DE CORPS À FORISOMES ARTIFICIELS POUR L'IMMOBILISATION D'ENZYMES OU LA PURIFICATION DE PROTÉINES RÉCOMBINANTES

(30) Priorität: 09.05.2012 EP 12167377
(43) Veröffentlichungstag der Anmeldung: 18.03.2015
(73) Patentinhaber: Fraunhofer Gesellschaft zur Förderung der angewandten Forschung e.V., 80686 München (DE)
(72) Erfinder: MÜLLER, Boje, 48155 Münster (DE); PRÜFER, Dirk, 48145 Münster (DE); FISCHER, Rainer, 52076 Aachen (DE)
(74) Vertreter: Schiweck Weinzierl Koch Patentanwälte Partnerschaft mbB
(86) Internationale Anmeldenummer: PCT/EP2013/059190
(87) Internationale Veröffentlichungsnummer: WO 2013/167471

(56) Entgegenhaltungen:
- BOJE MÜLLER ET AL: "Recombinant artificial forisomes provide ample quantities of smart biomaterials for use in technical devices", APPLIED MICROBIOLOGY AND BIOTECHNOLOGY, SPRINGER, BERLIN, DE, Bd. 88, Nr. 3, 28. Juli 2010 (2010-07-28), Seiten 689-698, XP019841768, ISSN: 1432-0614 in der Anmeldung erwähnt
- GUNDULA A. NOLL ET AL: "Characteristics of artificial forisomes from plants and yeast", BIOENGINEERED BUGS, Bd. 2, Nr. 2, 1. März 2011 (2011-03-01), Seiten 111-114, XP055032858, ISSN: 1949-1018, DOI: 10.4161/bbug.2.2.14368 in der Anmeldung erwähnt
- H. C. PELISSIER ET AL: "GFP Tagging of Sieve Element Occlusion (SEO) Proteins Results in Green Fluorescent Forisomes", PLANT AND CELL PHYSIOLOGY, Bd. 49, Nr. 11, 10. September 2008 (2008-09-10), Seiten 1699-1710, XP055032862, ISSN: 0032-0781, DOI: 10.1093/pcp/pcn141 in der Anmeldung erwähnt
- RÜPING BORIS ET AL: "Molecular and phylogenetic characterization of the sieve element occlusion gene family in Fabaceae and non-Fabaceae plants", BMC PLANT BIOLOGY, BIOMED CENTRAL, LONDON, GB, Bd. 10, Nr. 1, 8. Oktober 2010 (2010-10-08), Seite 219, XP021073732, ISSN: 1471-2229, DOI: 10.1186/1471-2229-10-219
- AMY SHEN: "Forisome as biomimetic smart materials", PROCEEDINGS OF SPIE, Bd. 5765, 7. März 2005 (2005-03-07), Seiten 97-107, XP055032871, ISSN: 0277-786X, DOI: 10.1117/12.606602
- SIRA GROSCURTH ET AL: "Artificial Forisomes Are Ideal Models of Forisome Assembly and Activity That Allow the Development of Technical Devices", BIOMACROMOLECULES, Bd. 13, Nr. 10, 8. Oktober 2012 (2012-10-08), Seiten 3076-3086, XP055078365, ISSN: 1525-7797, DOI: 10.1021/bm3008499
- CAMPBELL R. E. ET AL.: "A monomeric red fluorescent protein", PNAS, vol. 99, no. 12, 11 June 2002 (2002-06-11) , pages 7877-7882,

### SEQUENCE LISTING

<110> Fraunhofer-Gesellschaft zur Foerderung der angewandten Forschung e.V.
<120> Artifizielle Forisomenkrper mit SEO-F-Fusionsproteinen, pflanzliche oder Hefezellen mit Vektoren, die fr diese Proteine codieren, sowie Vektoren, die fr SEO-F-Fusionsproteine codieren
<130> 20046WO
<160> 10
<170> BiSSAP 1.0
<210> 1
   <211> 647
   <212> PRT
   <213> Medicago truncatula
<220>
   <221> SOURCE
   <222> 1..647
   <223> /mol_type="protein" /note="MtSEO-F1" /organism="Medicago truncatula"
<400> 1
<210> 2
   <211> 675
   <212> PRT
   <213> Medicago truncatula
<220>
   <221> SOURCE
   <222> 1..675
   <223> /mol_type="protein" /note="MtSEO-F2" /organism="Medicago truncatula"
<400> 2
<210> 3
   <211> 701
   <212> PRT
   <213> Medicago truncatula
<220>
   <221> SOURCE
   <222> 1..701
   <223> /mol_type="protein" /note="MtSEO-F3" /organism="Medicago truncatula"
<400> 3
   Met Ser Ser Ser Met Ala Pro Ser Ser Leu Val Ser Asn Val Ser Ala
   1               5                   10                  15
   Tyr Ser Gln Gln Ala Arg Thr Ser Asn Pro Leu Ala Trp Ser Asp Asp
               20                  25                  30
   Lys Ile Leu Glu Thr Val Tyr Leu Thr His Val His Thr Gly Glu Arg
           35                  40                  45
   Tyr Asp Val Glu Ser Leu Phe Asn Leu Thr Ser Asn Ile Leu Lys Arg
       50                  55                  60
   Ser Thr Ala Val Ala Asp Ser Val Ala Ser Lys Thr Gly Thr Pro Val
   65                  70                  75                  80
   Gly Leu Val Glu Asp Arg Leu Pro Leu Ser Gly Tyr Glu Pro Pro Ile
                   85                  90                  95
   Arg Lys Leu Lys His Ile Ser Ala Gln Met Met Ser Thr Leu Pro Gly
               100                 105                 110
   Glu His His Ala His Met Thr Thr Met Ser Ile Leu Asp Gln Leu Lys
           115                 120                 125
   Ser His Thr Trp Asp Gly Lys Ala Ile Phe Ala Leu Ala Ala Phe Ser
       130                 135                 140
   Leu Glu Tyr Gly Asn Phe Trp His Leu Val Gln Thr Pro Ser Gly Asp
   145                 150                 155                 160
   Thr Leu Gly Arg Ser Leu Ala Thr Met Asn Arg Val Gln Ser Val Asp
                   165                 170                 175
   Lys Asn Arg Gln Ala Ile Ala Asp Tyr Asn Ser Leu Val Lys Asn Leu
               180                 185                 190
   Leu Phe Ala Val Glu Cys Ile Thr Glu Leu Glu Lys Leu Ser Thr Lys
           195                 200                 205
   Gly Tyr Glu His Lys Asp Val Pro Ala Leu Ser Glu Ala Met Gln Glu
       210                 215                 220
   Ile Pro Val Ala Val Tyr Trp Ala Ile Ile Thr Ala Ile Ile Cys Ala
   225                 230                 235                 240
   Asn His Leu Asp Leu Leu Phe Gly Asp Ser Asp Asp Arg Tyr Glu Leu
                   245                 250                 255
   Ser Ser Tyr Asp Val Lys Leu Ala Ser Ile Val Ser Lys Leu Lys Ala
               260                 265                 270
   His Leu Thr Arg Ser Arg Lys His Ile Gly Glu Leu Glu Asp Tyr Trp
           275                 280                 285
   Arg Arg Lys Arg Val Leu Gln Thr Pro Thr Glu Ile Val Glu Val Leu
       290                 295                 300
   Lys Val Leu Val Phe His Asn Glu Ile Gln Asp Pro Leu Val Phe Asp
   305                 310                 315                 320
   Gly Leu Asn Arg Gln Met Val Ser Ile Glu Val Phe Arg Lys Lys His
                   325                 330                 335
   Val Leu Val Phe Ile Ser Gly Leu Asp Ser Ile Arg Asp Glu Ile Arg
               340                 345                 350
   Leu Leu Gln Ser Ile Tyr Val Gly Leu Gln Glu Glu Pro Arg Glu Leu
           355                 360                 365
   Lys Gly Tyr Arg Lys Glu Asp Phe Lys Ile Leu Trp Ile Pro Ile Val
       370                 375                 380
   Asp Asp Trp Thr Leu Leu His Lys Ala Glu Phe Asp Asn Leu Lys Leu
   385                 390                 395                 400
   Glu Met Pro Trp Tyr Val Val Glu Tyr Phe Tyr Pro Leu Ala Gly Ile
                   405                 410                 415
   Arg Leu Ile Arg Glu Asp Leu Ser Tyr Lys Asn Lys Pro Ile Leu Pro
               420                 425                 430
   Val Leu Asn Pro Leu Gly Arg Ile Val Asn His Asn Ala Met His Met
           435                 440                 445
   Ile Phe Val Trp Gly Ile Asp Ala Phe Pro Phe Arg Pro Thr Asp Asp
       450                 455                 460
   Glu Ser Leu Thr Gln Lys Trp Asn Trp Phe Trp Ala Glu Met Lys Lys
   465                 470                 475                 480
   Val Tyr Pro Arg Leu Gln Asp Leu Ile Lys Gly Asp Thr Phe Ile Phe
                   485                 490                 495
   Ile Tyr Gly Gly Thr Asp Pro Lys Trp Thr Gln Asp Phe Ala Leu Ala
               500                 505                 510
   Ile Glu Lys Ile Lys Arg His Glu Ile Thr Arg Lys Ala Asp Ala Val
           515                 520                 525
   Ile Glu His Phe His Phe Gly Lys Glu Asp Lys Arg Ile Val Pro Arg
       530                 535                 540
   Phe Trp Ile Gly Ile Glu Ser Leu Phe Ala Asn Met Ile Gln Lys Lys
   545                 550                 555                 560
   His Lys Asp Pro Thr Ile Asp Glu Ile Lys Ser Leu Leu Cys Leu Lys
                   565                 570                 575
   Gln Asp Gln Pro Gly Trp Val Leu Leu Ser Lys Gly Pro Asn Val Lys
               580                 585                 590
   Leu Leu Gly Arg Gly Asp Gln Met Tyr Ala Thr Ala Val Asp Phe Glu
           595                 600                 605
   Ile Trp Lys Glu Lys Val Leu Glu Lys Ala Gly Phe Asp Val Ala Phe
       610                 615                 620
   Lys Glu Tyr Tyr Glu Arg Lys Arg Arg Glu Tyr Pro Val Ala Cys Ala
   625                 630                 635                 640
   Asn Met Gln Leu Ala Asn Tyr Pro Ser Asp Ile Leu Asp Pro Ile Tyr
                   645                 650                 655
   Cys Pro Asp Ser Asn Cys Gly Arg Ser Met Glu Ile Ala Ser Val Ser
               660                 665                 670
   Tyr Lys Cys Cys His Gly His Thr His Glu Asn Ala Glu Val Ala Pro
           675                 680                 685
   Ala Glu Ser Gly Gly Phe Val Gln Ile Glu Lys Arg Ser
       690                 695                 700
<210> 4
   <211> 671
   <212> PRT
   <213> Medicago truncatula
<220>
   <221> SOURCE
   <222> 1..671
   <223> /mol_type="protein" /note="MtSEO-F4" /organism="Medicago truncatula"
<400> 4
   Met Ser Leu Ser Asn Leu Gly Ser Ala Thr Ala Thr Asn Ser Ser Leu
   1               5                   10                  15
   Asn Gln Lys Asn Ala Thr Asn Ser Leu Gln Asn Lys Ala Asn Phe Leu
               20                  25                  30
   Pro Asn Pro Phe Asp Leu His Asp Pro Gln Ile Leu Asp Arg Val Tyr
           35                  40                  45
   Leu Thr His Val Thr Asp Asp Glu Phe Cys Asp Thr Asn Ile Ile Phe
       50                  55                  60
   Glu Leu Val Ser Ser Val Val Leu Gln Thr Ile Pro Lys Ile Ser Val
   65                  70                  75                  80
   Thr Ser Phe Lys Pro Glu Phe Pro Thr Leu Lys Leu Ile Ser Cys Gln
                   85                  90                  95
   Met Ile Thr Thr Arg Asn Asp Pro His Cys Val His Gln Thr Thr Leu
               100                 105                 110
   Trp Ile Leu Gln Asn Leu Arg Ser Tyr Ser Trp Asp Ala Lys Ala Leu
           115                 120                 125
   Ile Thr Leu Ala Ala Phe Thr Leu Glu Tyr Gly Asn Tyr Leu Gln Leu
       130                 135                 140
   Asn Arg Val Thr Thr Thr Asp Thr Leu Gly Asn Ser Leu Arg Val Leu
   145                 150                 155                 160
   Asn Gln Val Gln Thr Arg Lys Ile Ser Asn Asp Val Thr Glu Leu Val
                   165                 170                 175
   Lys Tyr Ile Val Asp Met Leu Ile His Leu Asn Val Trp Ala Thr Trp
               180                 185                 190
   Ser Ala Asp Gly Tyr Asp Pro Val Asp Val Pro Ala Leu Thr Asp Ala
           195                 200                 205
   Leu Gln Glu Ile Pro Val Phe Val Tyr Trp Thr Ile Ala Ser Ile Val
       210                 215                 220
   Ala Ser Thr Gly Asn Leu Val Gly Val Ser Asp Tyr Lys Leu Ser Ala
   225                 230                 235                 240
   Tyr Lys Glu Arg Leu Ser Arg Val Val Glu Glu Leu Val Lys His Leu
                   245                 250                 255
   Ala Thr Cys Glu Arg Gln Ile Arg Asn Val Asp Asp Leu Thr Ser Arg
               260                 265                 270
   Thr Asn Asn Tyr Arg Lys Pro Lys Asp Ile Val Asp Cys Leu Lys Ala
           275                 280                 285
   Leu Ile His Arg Asn Gly Thr Asp Ile Pro Gln Ile Tyr Gln Gly Asn
       290                 295                 300
   Val Gln Val Lys Ser Gly Leu Asp Ile Phe Lys Gln Lys His Val Leu
   305                 310                 315                 320
   Leu Phe Ile Ser Ser Leu Asp Arg Ile Gln Asp Glu Ile Thr Leu Leu
                   325                 330                 335
   Asn Ser Ile Tyr Glu Arg Leu Gln Glu Asn Pro Lys Glu Ser Lys Gly
               340                 345                 350
   Phe Met Lys Glu Asp Phe Lys Ile Leu Trp Ile Pro Ile Val Lys Lys
           355                 360                 365
   Trp Asp Asp Ile Gln Ile Glu Asn Phe Lys Ala Leu Lys Ser Gly Ile
       370                 375                 380
   Lys Trp Tyr Val Val Glu Tyr Phe Ser Glu Leu Pro Gly Leu Lys Ile
   385                 390                 395                 400
   Ile Lys Asp Pro Glu Leu Ile Gly Tyr Ile Asp Asn Pro Ile Ile Pro
                   405                 410                 415
   Val Phe Asn Pro Lys Gly Ile Ile Thr Asn Glu Asp Ala Met Asp Leu
               420                 425                 430
   Ile Phe Gln Trp Gly Ile Asp Ala Phe Pro Phe Arg Lys Ser Asp Gly
           435                 440                 445
   Asn Asp Leu Lys Leu Lys Trp Asn Trp Leu Trp Asp Val Ile Lys Lys
       450                 455                 460
   Ala Thr Pro Gly Leu Leu Val Lys Val Asp Arg Tyr Ile Phe Ile Tyr
   465                 470                 475                 480
   Gly Gly Thr Asn Lys Lys Trp Ile Gln Asp Phe Thr Leu Glu Leu Glu
                   485                 490                 495
   Lys Ile Lys Arg His Glu Thr Ile Lys Arg Ala Asp Val Ile Ile Glu
               500                 505                 510
   Asn Tyr Gln Val Gly Lys Asp Asp Pro Asn Arg Val Pro Ser Phe Trp
           515                 520                 525
   Met Gly Ile Glu Arg Lys Lys Gln Asn Lys Lys His Gln Glu Thr Val
       530                 535                 540
   Asp Cys Lys Ile Gln Glu Ile Val Lys Asp Leu Phe Cys Leu Arg Arg
   545                 550                 555                 560
   Asp Pro Gln Gly Trp Ile Ile Leu Ser Lys Gly His Ser Ile Lys Leu
                   565                 570                 575
   Leu Gly His Gly Glu Pro Ala Tyr Gln Thr Leu Val Glu Phe Gln Asn
               580                 585                 590
   Trp Lys Asp Lys Val Leu Glu Lys Glu Gly Phe Asp Ile Ala Phe Lys
           595                 600                 605
   Glu Tyr Tyr Gln Met Lys Ala Lys Glu Ile Ser Gly Arg Glu Pro Cys
       610                 615                 620
   Glu Val Leu Asn Val Asp Thr Tyr Ser Ser Asn Val Ile Gly Thr Ile
   625                 630                 635                     640
   Ser Cys Pro Asn Pro Met Cys Gly Arg Val Met Glu Val Ser Ser Ile
                   645                 650                 655
   His Tyr Lys Cys Cys His Arg Asp Glu Pro Asn Asn Leu Gly Val
               660                 665                 670
<210> 5
   <211> 651
   <212> PRT
   <213> Dipteryx panamensis
<220>
   <221> SOURCE
   <222> 1..651
   <223> /mol_type="protein" /organism="Dipteryx panamensis"
<400> 5
   Met Ser Leu Ser Asn Gly Ala Ser Ser Thr Thr Leu Ser Gln Gln Lys
   1               5                   10                  15
   Thr Gln Leu Pro Asn Pro Phe Asp Leu Thr Asp Ser Gln Ile Leu Asp
               20                  25                  30
   Lys Val Tyr Leu Ser His Ala His Asp Asp Glu Glu Cys Asp Arg Asp
           35                  40                  45
   Thr Leu Leu Asp Leu Val Ser Ile Ile Ile Leu Lys Ser Gln Arg Pro
       50                  55                  60
   Ile Pro Leu Ala Lys Tyr Lys Pro Glu Phe Pro Thr Leu Lys Leu Ile
   65                  70                  75                  80
   Ser Cys Gln Met Ile Thr Thr Arg Gly Val Val His Cys Ala His Gln
                   85                  90                  95
   Thr Thr Met Trp Ile Leu Gln His Leu Arg Ser Phe Ser Trp Asp Ala
               100                 105                 110
   Lys Ala Leu Ile Thr Val Ala Ala Phe Ser Leu Glu Tyr Gly Asn Phe
           115                 120                 125
   Arg His Leu Gln Ile Pro Thr Ser Asp Gln Leu Gly Asn Ala Leu Lys
       130                 135                 140
   Gln Leu Asn Gln Val Asn Asn Gly Lys Leu Ser Asp Asp Ile Thr Glu
   145                 150                 155                 160
   Leu Ala Thr Val Thr Val Arg Val Leu Gln His Leu Lys Glu Trp Ala
                   165                 170                 175
   Ala Trp Ser Ala Ala Gly Tyr Asp Thr Glu Asp Val Pro Ala Leu Ser
               180                 185                 190
   Asp Ala Leu Gln Val Ile Pro Phe Val Val Tyr Trp Thr Ile Ala Ser
           195                 200                 205
   Ile Val Ala Ser Thr Gly Asn Leu Ile Gly Val Ser Asp Tyr Lys Leu
       210                 215                 220
   Ser Asp Phe Lys Asp Lys Leu Asp Arg Val Val Lys Thr Leu Asn Asp
   225                 230                 235                 240
   His Leu Asp Glu Cys Lys Lys Gln Ile Asp Val Ile Asp Asn Tyr Asn
                   245                 250                 255
   Trp Arg Arg Lys Ala Phe Glu Asn Pro Lys Asp Ile Val Asp Leu Leu
               260                 265                 270
   Lys Leu Leu Ile His Ser Lys Gly Ser Pro Ile Pro Gln Ile Tyr Asp
           275                 280                 285
   Gly Arg Thr Thr Thr Lys Thr Asp Ile Glu Val Phe Lys Gln Lys Tyr
       290                 295                 300
   Val Leu Leu Phe Ile Ser Ser Leu Asp Ser Ile Asp Asp Glu Ile Arg
   305                 310                 315                 320
   Leu Leu Asn Ser Ile Tyr Asp Arg Leu Lys Glu Asp Pro Lys Glu Val
                   325                 330                 335
   Lys Gly Phe Asn Lys Glu Asp Phe Lys Ile Leu Trp Ile Pro Ile Val
               340                 345                 350
   Asp Ser Trp Asp Lys Asp Ser Val Glu Lys Tyr Lys Thr Leu Lys Thr
           355                 360                 365
   Lys Ile Lys Trp Tyr Ala Val Glu Phe Leu Ser Leu Val Pro Gly Ile
       370                 375                 380
   Arg Leu Val Arg Glu Val Leu Lys Phe Glu Thr Lys Pro Ile Ile Pro
   385                 390                 395                 400
   Val Ile Ser Pro Gln Gly Lys Arg Ile Asn Asp Asn Ala Met Asp Ile
                   405                 410                 415
   Ile Phe Glu Trp Gly Val Asp Ala Phe Pro Phe Arg Lys Glu Asp Gly
               420                 425                 430
   Asp Gln Leu Thr Gln Lys Trp Lys Trp Phe Trp Asp Val Ile Lys Lys
           435                 440                 445
   Val Asn Pro Ala Ile Gln Val Glu Pro Glu Ser Tyr Ile Phe Ile Tyr
       450                 455                 460
   Gly Gly Thr Asp Asn Lys Trp Ile Gln Asp Phe Thr Leu Ala Val Asp
   465                 470                 475                 480
   Lys Val Lys Arg His Asp Thr Ile Lys Arg Ala Asp Ala Ile Ile Glu
                   485                 490                 495
   His His Gln Leu Ala Lys Asp Asp Ser Ile Val Pro Arg Phe Trp Ile
               500                 505                 510
   Gly Ile Glu Ser Lys Thr His Lys Lys His Gln Glu Ala Val Asp Cys
           515                 520                 525
   Gln Ile Gln Thr Ile Val Lys Ser Leu Leu Cys Leu Lys Arg Asp Pro
       530                 535                 540
   Gln Gly Trp Ala Ile Leu Ser Lys Gly Asn Asn Val Lys Ile Leu Gly
   545                 550                 555                 560
   His Gly Glu Pro Met Leu Gln Thr Leu Thr Gln Phe Glu Ser Trp Lys
                   565                 570                 575
   Asp Lys Val Leu Glu Lys Glu Gly Phe Asp Ile Ala Leu Lys Glu Phe
               580                 585                 590
   Tyr Asp Gly Lys Val Glu Ser Leu Ser Tyr Arg Gln Pro Cys Glu Tyr
           595                 600                 605
   Leu Asn Ile Asp Ser Gln Ser Ser Ser Val Ile Ala Thr Ile Thr Cys
       610                 615                620
   Pro Asn Pro Thr Cys Gly Arg Val Met Glu Val Thr Ser Val Asn Tyr
   625                 630                635                 640
   Arg Cys Cys His Arg Asp Gly Gln Lys Ile Cys
                   645                650
<210> 6
   <211> 668
   <212> PRT
   <213> Lotus japonicus
<220>
   <221> SOURCE
   <222> 1..668
   <223> /mol_type="protein" /organism="Lotus japonicus"
<400> 6
   Met Ser His Val Pro Lys Ala Ala Ser Asn Gly Ala Leu Ile Gln His
   1               5                   10                  15
   Ser Gly Thr Ser Pro Asn Gln Lys Ala Tyr Leu Pro Ser Pro Phe Glu
               20                  25                  30
   Leu Lys Asp Pro Gln Ile Leu Asp Arg Val Tyr Leu Thr His Val Asn
           35                  40                  45
   Asp Asp Glu Ile Cys Asp Thr Lys Ile Leu Phe Asp Leu Val Ser Thr
       50                  55                  60
   Val Val Leu Gln Ser Val Ser Gln Ile Pro Ala Thr Ser Phe Lys Pro
   65                  70                  75                  80
   Glu Phe Ser Thr Leu Lys Leu Ile Ser Cys Gln Met Ile Thr Thr Arg
                   85                  90                  95
   Asn Ala Asp His Cys Val His Gln Thr Thr Met Trp Ile Leu Gln Asn
               100                 105                 110
   Leu Arg Ser Tyr Ser Trp Asp Ala Lys Ala Ile Ile Thr Leu Ala Ala
           115                 120                 125
   Phe Thr Leu Glu Tyr Gly Asn Tyr Leu His Leu Ser Arg Ala Ala Val
       130                 135                 140
   Ala Asp Thr Leu Gly Ser Ser Leu Arg Gln Leu Asn Gln Val His Thr
   145                 150                 155                 160
   Arg Lys Val Pro Ala Asp Ile Thr Lys Leu Val Thr Phe Ile Val His
                   165                 170                 175
   Ala Phe Gln His Leu Lys Glu Trp Ala Thr Trp Ala Asp Glu Gly Tyr
               180                 185                 190
   Glu Pro Glu Glu Val Pro Ser Leu Thr Glu Ala Leu Gln His Val Pro
           195                 200                 205
   Val Ala Val Tyr Trp Thr Ile Ala Ala Ile Val Ala Ser Thr Gly Asn
       210                 215                 220
   Leu Val Gly Val Ser Thr Tyr Asn Leu Gln Gly Tyr Ile Asp Arg Leu
   225                 230                 235                 240
   Asp Glu His Val Thr Lys Leu Ala Glu Gln Leu Asn Ser Cys Lys Leu
                   245                 250                 255
   Gln Ile Gly His Val Asp Asp Tyr Phe Asn Arg Arg Lys Ile Phe Asp
               260                 265                 270
   Lys Pro Lys Asp Ile Val Asp Leu Leu Lys Ala Leu Ile His Arg Asn
           275                 280                 285
   Gly Ala Gln Gly Pro Gln Ile Phe Glu Gly Gly Val Ile Val Lys Gln
       290                 295                 300
   Gly Leu Glu Val Phe Arg Gln Lys His Val Leu Leu Phe Ile Ser Gly
   305                 310                 315                 320
   Leu Asn Ser Ile Val Asp Glu Ile Leu Leu Leu Asn Ser Ile Tyr Asn
                   325                 330                 335
   Arg Leu Gln Asp Asn Pro Thr Glu Val Ile Lys Gly Phe Lys Lys Glu
               340                 345                 350
   Asp Phe Lys Ile Leu Trp Val Pro Met Val Asp Arg Trp Asp Glu Ala
           355                 360                 365
   Ser Arg Glu Gln Tyr Leu Asn Thr Trp Lys Arg Gly Ile Lys Trp Tyr
       370                 375                 380
   Ile Val Glu Tyr Phe Phe Glu Leu Pro Gly Arg Arg Ile Ile Thr Asp
   385                 390                 395                 400
   Pro Glu Arg Leu Gly Tyr Glu Gly Asn Pro Ile Ile Pro Val Phe Asn
                   405                 410                 415
   Pro Gln Gly Met Leu Thr Asn Asp Asn Ala Met Asp Leu Ile Phe Gln
               420                 425                 430
   Trp Gly Ile Asp Ala Phe Pro Phe Arg Lys Ser Asp Gly Ile Asp Leu
           435                 440                 445
   Thr Leu Lys Trp Lys Trp Leu Trp Asp Ile Ile Lys Lys Ala Thr Pro
       450                 455                 460
   Gly Leu Gln Val Lys Val Asp Arg Tyr Ile Phe Ile Phe Gly Ser Thr
   465                 470                 475                 480
   Asn Asn Lys Trp Ile Gln Asp Phe Thr Ile Glu Leu Asp Lys Leu Lys
                   485                 490                 495
   Arg Asn Glu Thr Val Lys Arg Ala Asp Val Ile Ile Glu Gln Tyr Gln
               500                 505                 510
   Leu Gly Lys Asp Asp Pro Asn Arg Val Pro Ser Phe Trp Met Gly Val
           515                 520                 525
   Glu Arg Lys Lys Gln Asn Lys Lys His Gln Glu Ala Val Asp Cys Glu
       530                 535                 540
   Ile Gln Gly Ile Val Lys Ser Leu Phe Cys Leu Lys Arg Asp Pro Gln
   545                 550                 555                 560
   Gly Trp Val Ile Leu Ser Lys Gly His Asn Ile Lys Leu Leu Gly His
                   565                 570                 575
   Gly Glu Ala Val Tyr Gln Thr Val Val Glu Phe Pro Asn Trp Lys Glu
               580                 585                 590
   Lys Val Leu Glu Arg Glu Gly Phe Asp Ile Ala Phe Lys Glu Tyr Tyr
           595                 600                 605
   Asp Ile Lys Ala Lys Glu Ile Ser Ala Arg Gln Pro Cys Glu Ile Ile
       610                 615                 620
   Asn Val Asp Ser Tyr Ser Ala Asn Val Ile Ala Thr Ile Thr Cys Pro
   625                 630                 635                 640
   Asn Pro Met Cys Gly Arg Val Met Glu Val Thr Ser Val Asn Tyr Lys
                   645                 650                 655
   Cys Cys His Ser Asp Ala Pro Asn Gly Phe Gly Ile
   660 665
<210> 7
   <211> 685
   <212> PRT
   <213> Pisum sativum
<220>
   <221> SOURCE
   <222> 1..685
   <223> /mol_type="protein" /organism="Pisum sativum"
<400> 7
   Met Ser Phe Ser Asn Ser Ala Ala Ala Ala Thr Gly Thr Leu Val Gln
   1               5                   10                  15
   His Gly Gly Asn Ala Thr Asn Asn Asn Ser Leu Ile Gln Lys Asn Ala
               20                  25                  30
   Thr Ser Pro His Ser His His Lys Ala Asn Asn Tyr Leu Pro Asn Pro
           35                  40                  45
   Phe Glu Leu His Asp Ser Gln Ile Leu Asp Lys Val Tyr Leu Thr His
       50                  55                  60
   Val Thr Asp Asp Gln Phe Cys Asp Thr Asp Ile Ile Phe Asp Leu Val
   65                  70                  75                  80
   Ser Thr Leu Val Leu Gln Thr Asn Thr Gln Ile Pro Val Thr Gly Phe
                   85                  90                  95
   Lys Pro Asp Phe Pro Thr Leu Lys Leu Ile Ser Cys Gln Met Ile Thr
               100                 105                 110
   Thr Arg Ser Ala Ala His Cys Val His Gln Thr Thr Leu Trp Ile Leu
           115                 120                 125
   Gln Asn Leu Arg Ser Tyr Ser Trp Asp Ala Lys Ala Leu Ile Thr Leu
       130                 135                 140
   Ala Ala Phe Thr Leu Glu Tyr Gly Asn Tyr Leu His Leu Thr Arg Val
   145                 150                 155                 160
   Thr Ala Thr Asp Pro Ile Gly Asn Ser Leu Arg Gln Leu Asn Gln Ile
                   165                 170                 175
   Gln Thr Arg Asn Ile Ser Thr Asp Ile Thr Glu Leu Val Ser Phe Ile
               180                 185                 190
   Val His Gln Leu Leu His Leu Lys Glu Trp Ala Thr Trp Ser Ala Glu
           195                 200                 205
   Gly Tyr Asp Pro Glu Asp Val Pro Ala Leu Thr Glu Ala Leu Gln Glu
       210                 215                 220
   Ile Pro Val Phe Val Tyr Trp Thr Ile Ala Ser Ile Val Ala Ser Thr
   225                 230                 235                 240
   Gly Asn Leu Val Gly Val Ser Asp Tyr Lys Leu Ser Glu Tyr Arg Glu
                   245                 250                 255
   Arg Leu Ser Gly Ile Val Gln Lys Leu Val Val His Leu Asn Asn Cys
               260                 265                 270
   Lys Leu Gln Ile Ser Tyr Ile Asp Asp Leu Phe Asn Arg Lys Lys Ile
           275                 280                 285
   Phe Asp Lys Pro Lys Asp Ile Val Asp Cys Leu Lys Ala Leu Ile His
       290                 295                 300
   Arg Asn Gly Thr Asp Ser Pro Gln Ile Tyr Glu Gly Ala Ile His Val
   305                 310                 315                 320
   Lys Thr Gly Leu Glu Val Phe Arg Asn Lys His Val Leu Val Phe Ile
                   325                 330                 335
   Ser Ser Leu Asp Ser Ile Glu Asp Glu Ile Ser Leu Leu Asn Ser Ile
               340                 345                 350
   Tyr Glu Arg Leu Gln Glu Asn Ser Lys Glu Ser Ile Lys Gly Phe Lys
           355                 360                 365
   Lys Glu Asp Phe Lys Ile Leu Trp Ile Pro Ile Val Asn Asn Trp Asp
       370                 375                 380
   Asp Ile Arg Lys Glu Arg Phe Arg Ala Leu Lys Ser Gly Ile Lys Trp
   385                 390                 395                 400
   Tyr Ala Val Glu Tyr Phe Tyr Glu Leu Pro Gly His Arg Ile Ile Thr
                   405                 410                 415
   Asp Pro Glu Arg Ile Gly Tyr Ile Gly Asn Pro Ile Ile Pro Val Phe
               420                 425                 430
   Asn Pro Gln Gly Tyr Ile Thr Asn Ile Asp Ala Met Asp Leu Ile Phe
           435                 440                 445
   Gln Trp Gly Ile Asp Ala Phe Pro Phe Arg Lys Ser Asp Gly Ile Asp
       450                 455                 460
   Leu Thr Leu Lys Trp Lys Trp Leu Trp Asp Val Ile Lys Lys Ala Thr
   465                 470                 475                 480
   Pro Gly Leu Gln Val Lys Gly Asp Arg Tyr Ile Phe Ile Tyr Gly Gly
                   485                 490                 495
   Thr Asn Asn Lys Trp Ile Gln Asp Phe Thr Leu Glu Leu Glu Lys Ile
               500                 505                 510
   Lys Arg His Glu Ile Leu Lys Arg Ala Asp Val Ile Ile Glu Asn Tyr
           515                 520                 525
   Gln Leu Gly Lys Glu Asp Pro Asn Arg Val Pro Ser Phe Trp Ile Gly
       530                 535                 540
   Val Glu Arg Lys Lys Gln Asn Lys Lys His Gln Glu Ala Leu Asp Cys
   545                 550                 555                 560
   Glu Ile Gln Asp Ile Val Lys Ser Leu Phe Cys Leu Lys Arg Asp Pro
                   565                 570                 575
   Gln Gly Trp Ile Ile Leu Ser Lys Gly Gln Asn Ile Lys Leu Leu Gly
               580                 585                 590
   His Gly Glu Pro Ala Tyr Gln Thr Leu Ala Glu Phe Gln Asn Trp Lys
           595                 600                 605
   Asp Arg Val Leu Glu Lys Glu Gly Phe Asp Ile Ala Phe Lys Glu Tyr
       610                 615                 620
   Tyr Glu Met Lys Ala Lys Glu Leu Ser Gly Arg Gln Pro Cys Glu Val
   625                 630                 635                 640
   Val Asn Val Asp Thr Tyr Ser Ser Asn Val Ile Ala Thr Ile Ala Cys
                   645                 650                 655
   Pro Asn Pro Met Cys Gly Arg Val Met Glu Val Ser Ser Ala His Tyr
               660                 665                 670
   Lys Cys Cys His Arg Asp Glu Pro Asn Asn Phe Gly Val
   675 680 685
<210> 8
   <211> 684
   <212> PRT
   <213> Vicia faba
<220>
   <221> SOURCE
   <222> 1..684
   <223> /mol_type="protein" /organism="Vicia faba"
<400> 8
   Met Ser Phe Ser Asn Ser Pro Ala Ala Ala Thr Gly Thr Leu Val Gln
   1               5                   10                  15
   His Gly Gly Asn Gly Thr Asn Asn Ser Leu Ile Gln Lys Thr Ala Thr
               20                  25                  30
   Ser Ser His Pro His His Lys Ala Asn Asn Tyr Leu Pro Asn Pro Phe
           35                  40                  45
   Glu Leu His Asp Ser His Ile Leu Asp Lys Val Tyr Leu Thr His Val
       50                  55                  60
   Thr Asp Asp Glu Phe Cys Asp Thr Asp Ile Ile Phe Asp Leu Val Ser
   65                  70                  75                  80
   Thr Leu Ile Leu Gln Ser Asn Thr Gln Ile Pro Val Thr Gly Phe Lys
                   85                  90                  95
   Pro Asp Phe Pro Thr Leu Lys Leu Ile Ser Cys Gln Met Ile Thr Thr
               100                 105                 110
   Arg Ser Val Ala His Cys Val His Gln Thr Thr Leu Trp Ile Leu Gln
           115                 120                 125
   Asn Leu Arg Ser Tyr Ser Trp Asp Ala Lys Ala Leu Ile Thr Leu Ala
       130                 135                 140
   Ala Phe Thr Leu Glu Tyr Gly Asn Tyr Leu Gln Leu Asn Arg Val Thr
   145                 150                 155                 160
   Ala Thr Asp Pro Ile Gly Asn Ser Leu Arg Gln Leu Asn Gln Ile Gln
                   165                 170                 175
   Thr Arg Lys Ile Ser Thr Asp Ile Pro Glu Leu Val Asn Phe Ile Val
               180                 185                 190
   His Lys Leu Leu His Leu Lys Glu Trp Ala Ala Trp Ser Ala Glu Gly
           195                 200                 205
   Tyr Asp Pro Glu Asp Val Pro Ala Leu Thr Glu Ala Leu Gln Glu Ile
       210                 215                 220
   Pro Val Phe Val Tyr Trp Thr Ile Ala Ser Ile Val Ala Ser Thr Gly
   225                 230                 235                 240
   Asn Leu Val Gly Val Ser Asp Tyr Asn Leu Ser Glu Tyr Arg Glu Arg
                   245                 250                 255
   Leu Ser Gly Ile Val Gln Lys Leu Val Val His Leu Asn Asn Cys Lys
               260                 265                 270
   Leu Gln Ile Ser Tyr Ile Asp Asp Leu Phe Asn Arg Arg Lys Ile Phe
           275                 280                 285
   Asp Lys Pro Lys Asp Ile Val Asp Cys Leu Lys Ala Leu Ile His His
       290                 295                 300
   Asn Gly Ala Asp Ser Pro Gln Ile Tyr Glu Gly Ala Ile His Val Lys
   305                 310                 315                 320
   Thr Gly Leu Glu Val Phe Arg His Lys His Val Leu Met Phe Ile Ser
                   325                 330                 335
   Ser Leu Asp Ser Ile Glu Asp Glu Ile Ser Leu Leu Asn Ser Ile Tyr
               340                 345                 350
   Glu Arg Leu Gln Glu Asn Ser Lys Glu Ser Ile Lys Gly Phe Lys Lys
           355                 360                 365
   Glu Asp Phe Lys Ile Leu Trp Ile Pro Ile Val Asn Asn Trp Asp Asp
       370                 375                 380
   Ile Arg Lys Glu Arg Phe Arg Ala Leu Lys Ser Gly Ile Lys Trp Tyr
   385                 390                 395                 400
   Ala Val Glu Tyr Phe Tyr Glu Leu Pro Gly His Arg Ile Ile Thr Asp
                   405                 410                 415
   Pro Glu Arg Ile Gly Tyr Ile Gly Asn Pro Ile Ile Pro Val Phe Asn
               420                 425                 430
   Pro His Gly Tyr Ile Thr Asn Ile Asp Ala Met Asp Leu Ile Phe Gln
           435                 440                 445
   Trp Gly Ile Asp Ala Phe Pro Phe Arg Lys Ser Asp Gly Ile Asp Leu
       450                 455                 460
   Thr Phe Lys Trp Lys Trp Leu Trp Asp Val Ile Lys Lys Ala Thr Pro
   465                 470                 475                 480
   Gly Leu Gln Val Lys Gly Asp Arg Tyr Ile Phe Ile Tyr Gly Gly Thr
                   485                 490                 495
   Asn Asn Lys Trp Ile Gln Asp Phe Thr Leu Glu Leu Glu Lys Ile Lys
               500                 505                 510
   Arg His Glu Thr Leu Lys Arg Ala Asp Val Ile Ile Asp Asn Tyr Gln
           515                 520                 525
   Leu Gly Lys Asp Asp Pro Asn Arg Val Pro Ser Phe Trp Ile Gly Val
       530                 535                 540
   Glu Arg Lys Lys Gln Asn Lys Lys His Gln Glu Ala Val Asp Cys Glu
   545                 550                 555                 560
   Ile Gln Asp Ile Val Lys Ser Leu Phe Cys Leu Lys Arg Asp Pro Gln
                   565                 570                 575
   Gly Trp Val Ile Leu Ser Lys Gly Gln Asn Ile Lys Leu Leu Gly His
               580                 585                 590
   Gly Glu Pro Ala Tyr Gln Thr Leu Ala Glu Phe Gln Asn Trp Lys Asp
           595                 600                 605
   Arg Val Leu Glu Lys Glu Gly Phe Asp Ile Ala Phe Lys Glu Tyr Tyr
       610                 615                 620
   Glu Met Lys Ala Lys Glu Leu Ser Gly Arg Glu Pro Cys Glu Val Val
   625                 630                 635                 640
   Asn Val Asp Thr Tyr Ser Ser Asn Val Ile Ala Thr Ile Ala Cys Pro
                   645                 650                 655
   Asn Pro Met Cys Gly Arg Val Met Glu Val Ser Ser Val His Tyr Lys
               660                 665                 670
   Cys Cys His Arg Asp Glu Pro Asn Asn Phe Gly Val
           675                 680
<210> 9
   <211> 238
   <212> PRT
   <213> Aequorea victoria
<220>
   <221> SOURCE
   <222> 1..238
   <223> /mol_type="protein" /organism="Aequorea victoria"
<400> 9
   Met Ser Lys Gly Glu Glu Leu Phe Thr Gly Val Val Pro Ile Leu Val
   1               5                   10                  15
   Glu Leu Asp Gly Asp Val Asn Gly His Lys Phe Ser Val Ser Gly Glu
               20                  25                  30
   Gly Glu Gly Asp Ala Thr Tyr Gly Lys Leu Thr Leu Lys Phe Ile Cys
           35                  40                  45
   Thr Thr Gly Lys Leu Pro Val Pro Trp Pro Thr Leu Val Thr Thr Leu
       50                  55                  60
   Thr Tyr Gly Val Gln Cys Phe Ser Arg Tyr Pro Asp His Met Lys Gln
   65                  70                  75                  80
   His Asp Phe Phe Lys Ser Ala Met Pro Glu Gly Tyr Val Gln Glu Arg
                   85                  90                  95
   Thr Ile Phe Phe Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Tyr Asn Ser His Asn Val Tyr Ile Met Ala Asp Lys Gln Lys Asn Gly
   145                 150                 155                 160
   Ile Lys Val Asn Phe Lys Ile Arg His Asn Ile Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Lys
   225                 230                 235
<210> 10
   <211> 239
   <212> PRT
   <213> Venus
<220>
   <221> SOURCE
   <222> 1..239
   <223> /mol_type="protein" /organism="Venus"
<400> 10
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Leu Ile
           35                  40                  45
   Cys Thr Thr Gly Lys Leu Pro Val Pro Trp Pro Thr Leu Val Thr Thr
       50                  55                  60
   Phe Gly Tyr Gly Leu Gln Cys Phe Ala Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Thr Ala Asp Lys Gln Lys Asn
   145                 150                 155                 160
   Gly Ile Lys Ala Asn Phe Lys Ile Arg His Asn Ile Glu Asp Gly Gly
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Tyr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235

## Beschreibung

Die vorliegende Erfindung betrifft die Verwendung artifizieller Forisomenkörper zur Enzymimmobilisierung oder zur Aufreiningung rekombinanter Proteine mit proteinchemisch nutzbaren Eigenschaften. Ferner sind hierin pflanzliche Zellen und Hefezellen mit einer Kombination von Vektoren, die die Bildung solcher Forisomenkörper in der Zelle und deren Isolierung ermöglichen, sowie neue Vektoren, die für SEO-F-Fusionsproteine codieren, beschrieben.

Forisome sind pflanzliche Proteinkörper (Mechanoproteine), die sich ausschließlich im Phloem der Pflanzenfamilie der *Fabaceae* (Leguminosen) finden. Sie liegen in den Siebelementen des Leitgewebes vor. Wird das Phloem verletzt, werden die Forisomen einer calciumabhängigen Konformationsveränderung unterworfen, bei der sie von einem kondensierten in einen verdickten, dispergierten Zustand übergehen, durch den sie die Siebröhren verschließen, wodurch der Austritt wertvoller Zuckermoleküle verhindert wird. Forisome liegen in einer fibrillären Substruktur aus großen, kompakten Bündeln vor. Sie können in vitro durch divalente Kationen, pH-Veränderungen oder elektrische Stimuli ATP-unabhängig zu vielfach wiederholbaren Zyklen einer abwechselnden Kontraktion und Expansion angeregt werden.

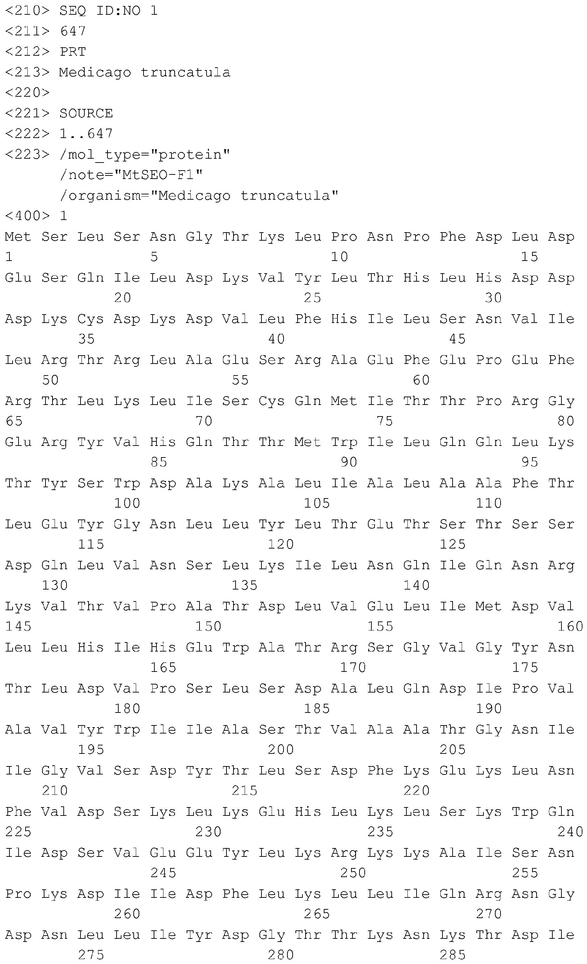

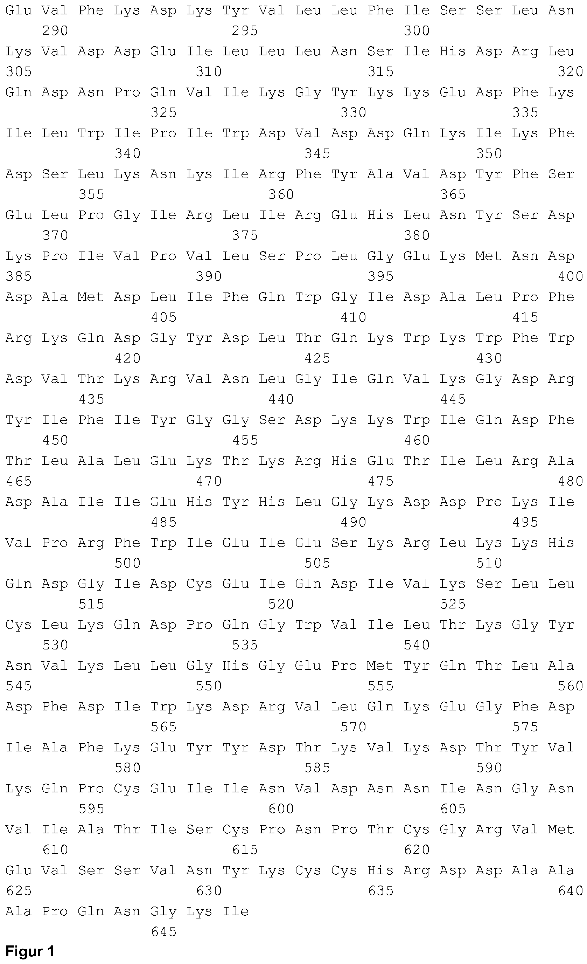

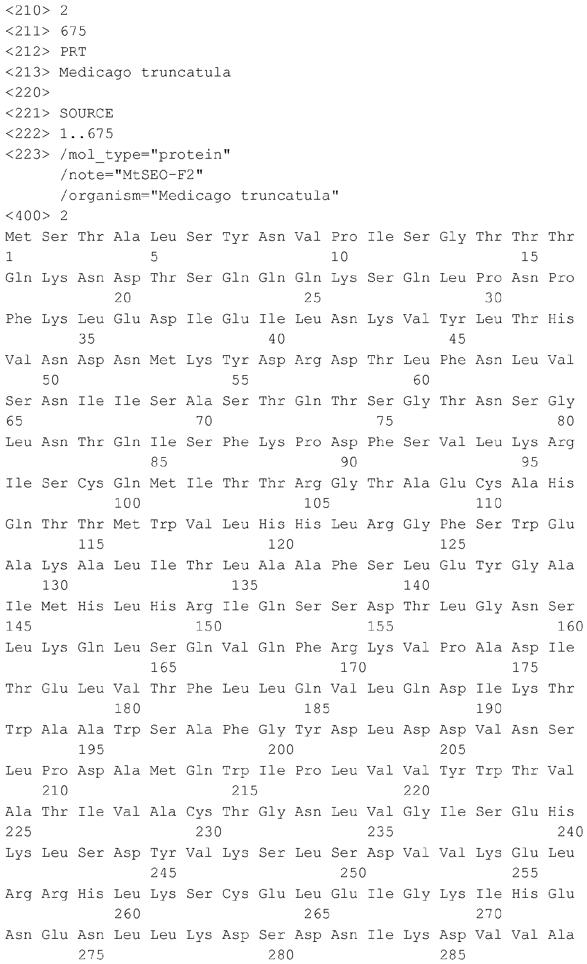

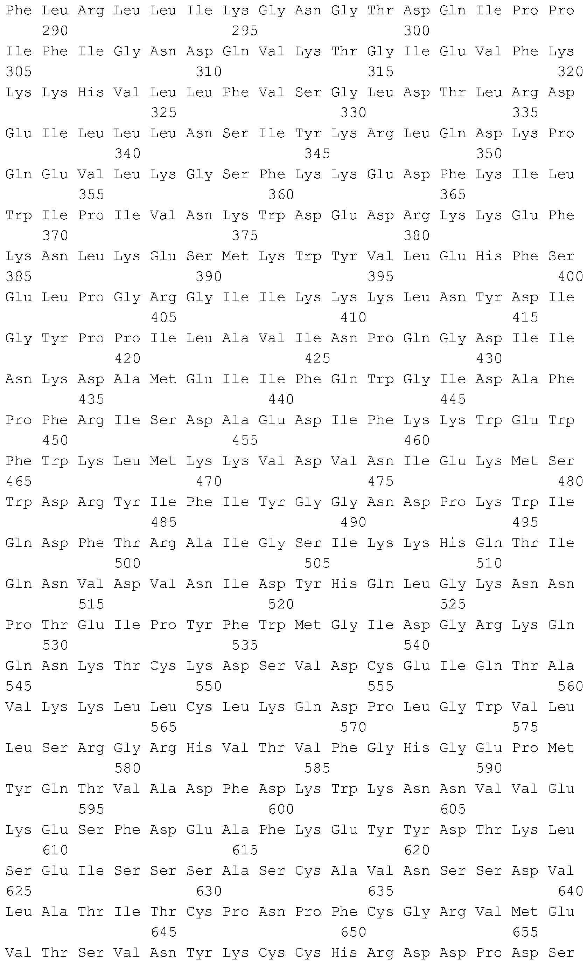

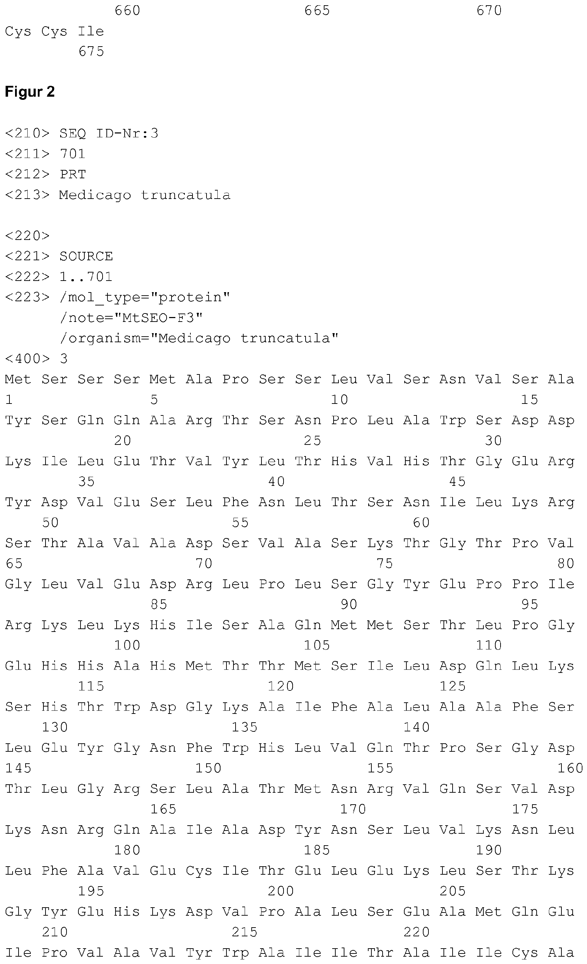

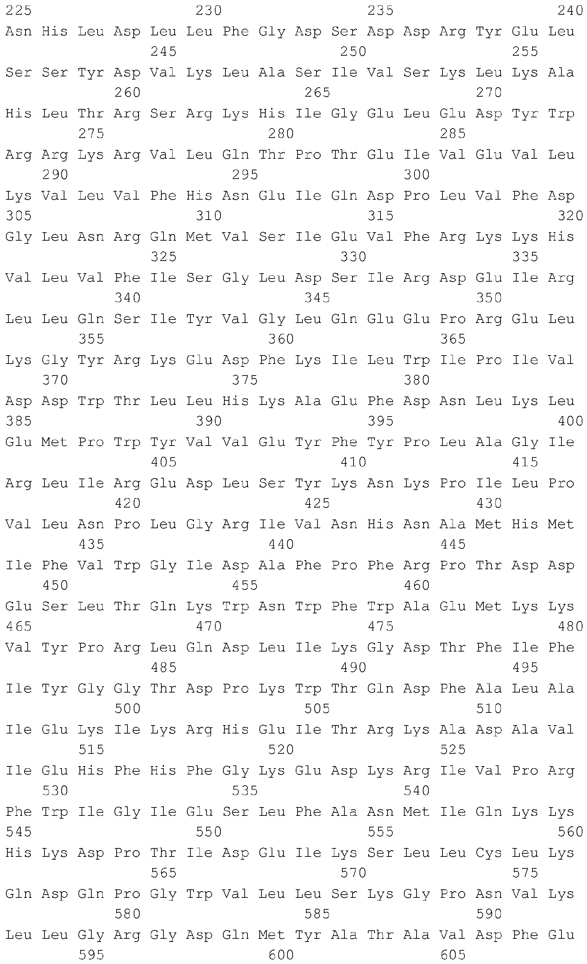

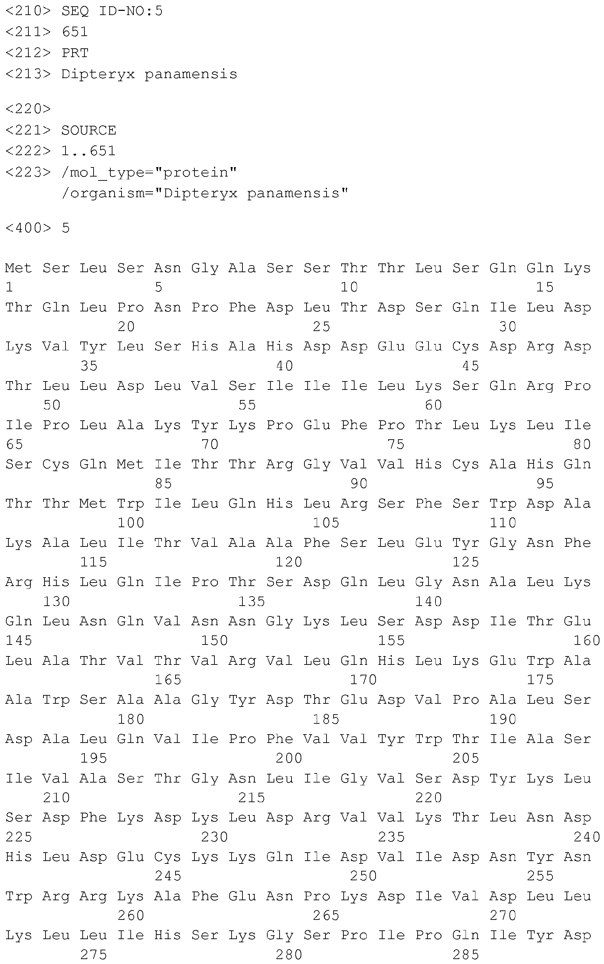

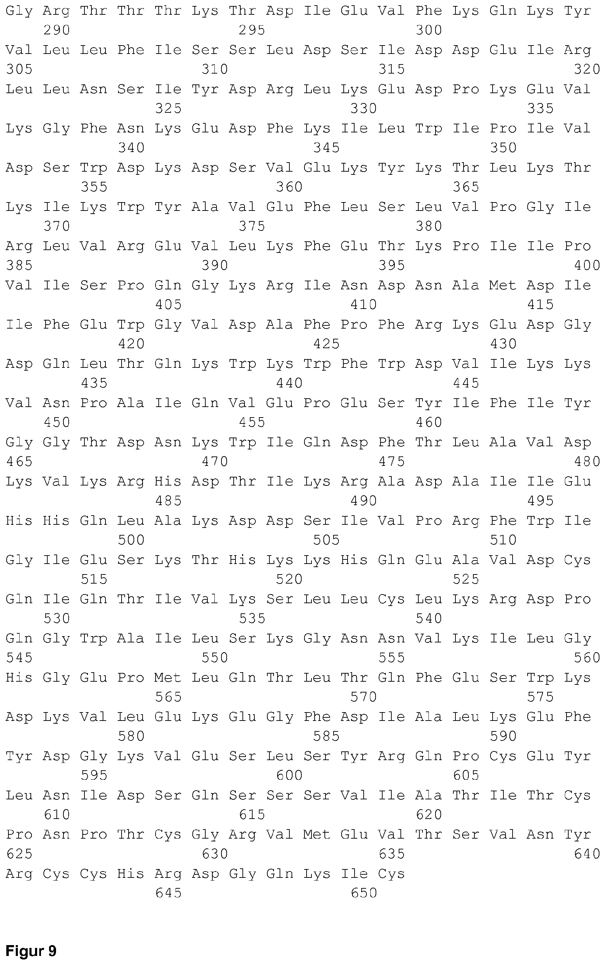

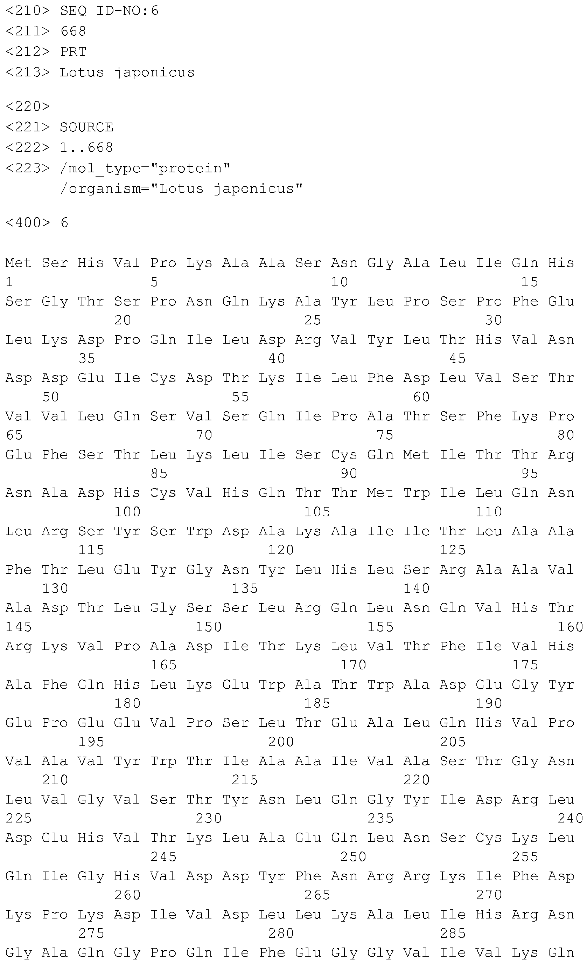

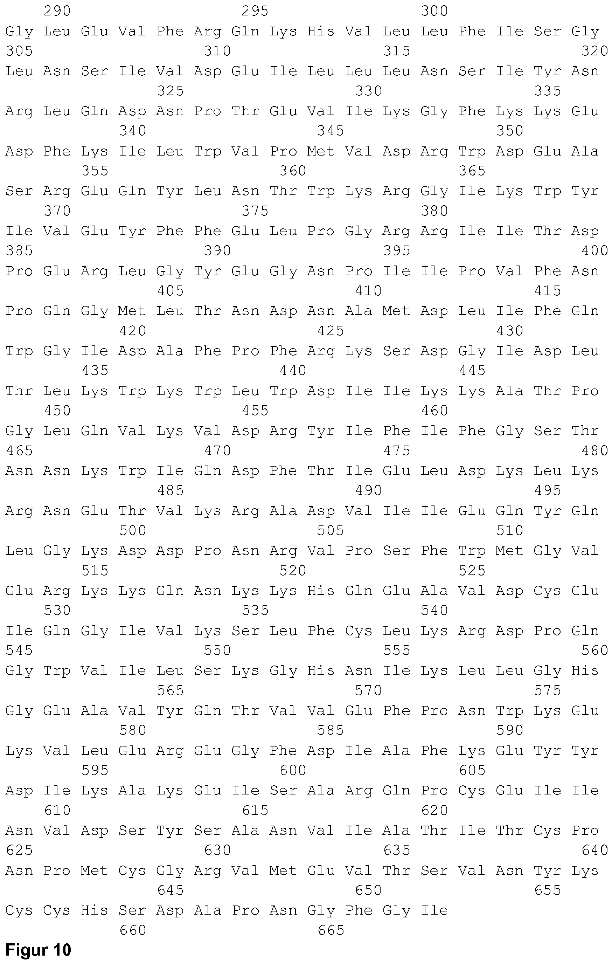

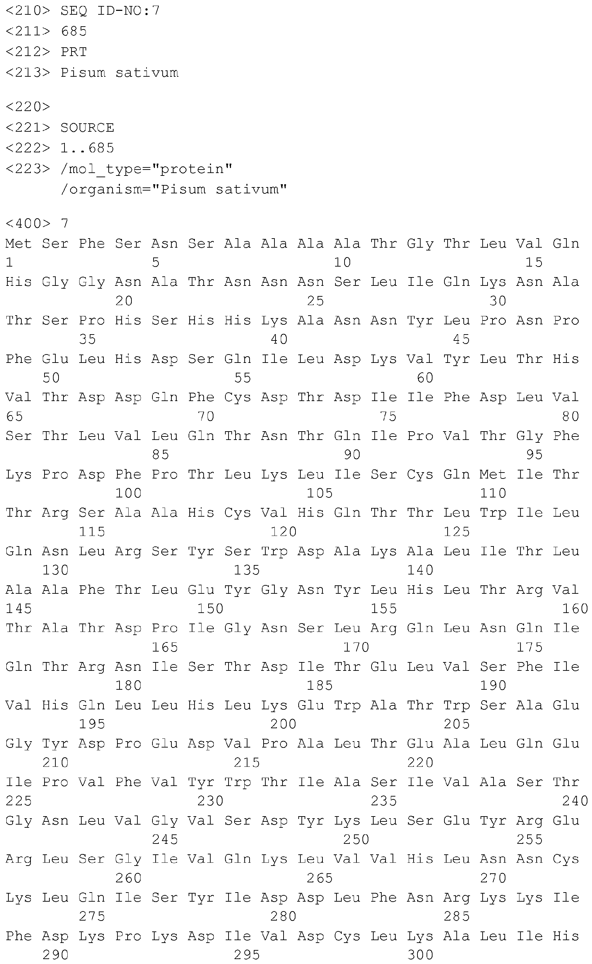

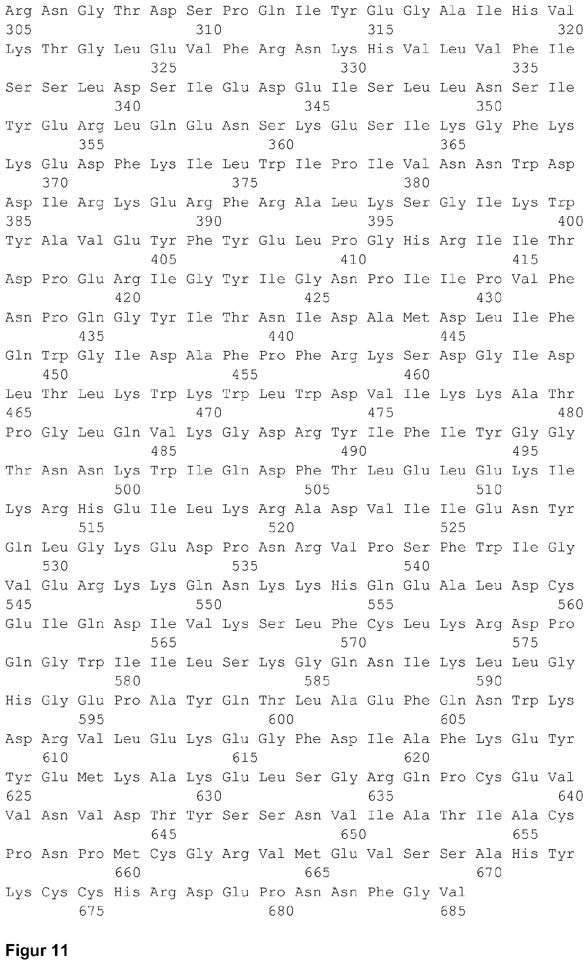

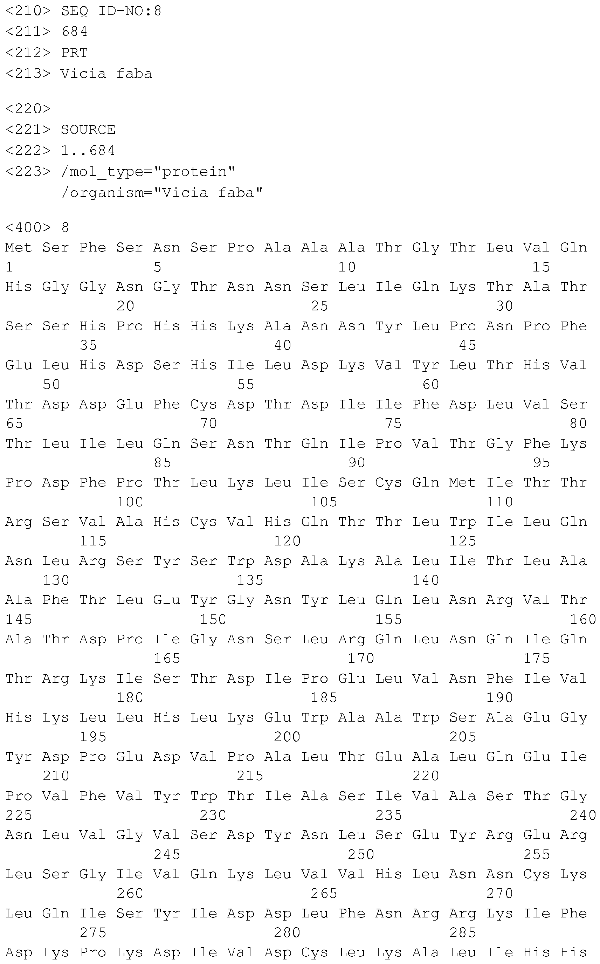

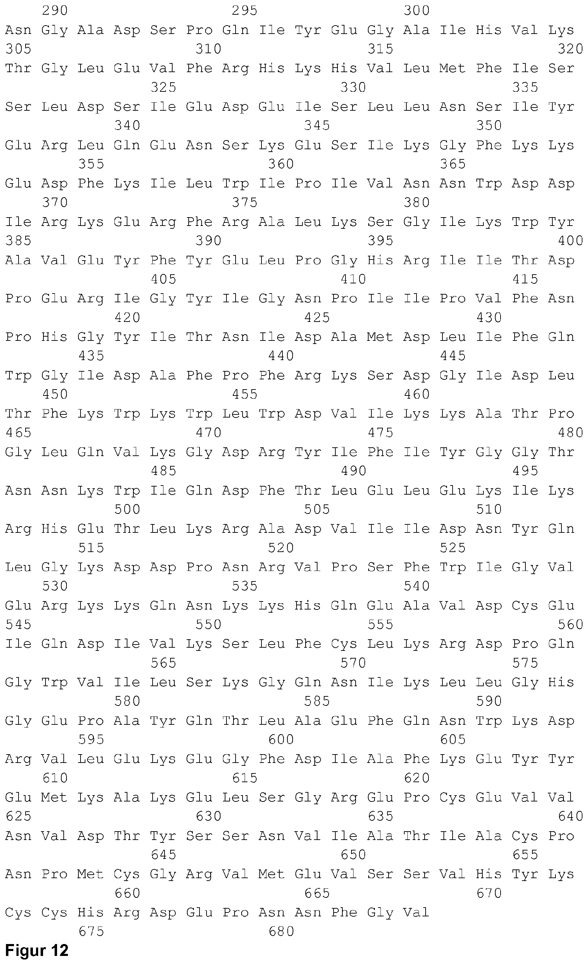

Ein Forisom ist aus mehreren Millionen Untereinheiten aufgebaut. Bei diesen Untereinheiten handelt es sich um homologe Proteine, die ihrer Funktion entsprechend mit "Sieve Element Occlusion by Forisomes" (SEO-F) benannt sind. Die Expression einiger Gene, die für diese Proteine codieren, mit Hilfe von bakteriellen Expressionsvektoren sind in der Dissertation von Gundula Noll (2005) beschrieben. Es konnte gesichert werden, dass es bei *Medicago trunculata* mindestens vier Untereinheiten (SEO-F1 bis SEO-F4) gibt (G. Noll et al., Plant Mol. Biol. 65:285-294 (2007), H.C. Pelissier et al., Plant Cell Physiol. 49:1699-1710 (2008)). Alle vier Untereinheiten wurden mittlerweile sequenziert; ihre Sequenzen (SEQ-ID NO: 1-4) sind in den **Figuren 1-4** dargestellt. Die Sequenzen der SEO-F1-Proteine der Species *Dipteryx panamensis, Lotus japonicus, Pisum sativum und Vicia Faba* (SEQ-ID NO: 5-8) sind in den **Figuren 9-12** dargestellt. In der Pflanze assemblieren die unterschiedlichen SEO-F-Proteine zum Forisomenproteinkörper. Mittels Expression der entsprechenden Gene in Fremdorganismen (Tabakpflanze, Hefe) konnte inzwischen gezeigt werden, dass sich bei *Medicago trunculata* zwei der Untereinheiten, nämlich SEO-F1 und SEO-F4, in Abwesenheit jeweils weiterer Untereinheiten zu homomeren artifiziellen Forisomen zusammenlagern, siehe G. Noll et al., Bioengineered Bugs 2:2, 1-4 (2011), 2011 Landes Bioscience. Die Untereinheit SEO-F2 dagegen kann sich nicht zu homomeren Forisomen zusammenlagern, koassembliert jedoch sowohl mit der Untereinheit SEO-F1 als auch mit der Untereinheit SEO-F4.

Für analytische Zwecke wurden vereinzelt bereits SEO-F-Fusionsproteine erzeugt. So führte G. Noll gemäß ihrer Dissertation (2005) eine Forisomengen-Enzymkopplung zum Zwecke der Antikörperproduktion in *E. coli* durch. Die Bildung von Forisomenkörpern konnte dabei jedoch nicht beobachtet werden. H. C. Pelissier *et al.* beschreiben a.a.O. ein Fusionsprotein aus einer Forisomen-Untereinheit und dem grün fluoreszierenden Protein (GFP), mit dem sie die Zugehörigkeit dieser Untereinheit zum Forisomenkörper in transgenen Pflanzen nachweisen konnten, in die das Fusionsprotein eingelagert war. B. Müller et al. beschreiben in Appl. Microbiol. Biotechnol. (2010) 88:689-698 (2010) die Herstellung von vier Vektoren, die für ein Fusionsprotein aus einem der Gene MtSEO1 bis MtSEO4 aus *Medicago truncatula* und dem *Venus-Gen* für ein gelb fluoreszierendes Protein codieren. Das Fusionsprotein konnte erfolgreich in Epidermis-Zellen von *N. benthamiana* exprimiert werden; wurde das jeweilige MtSEO-Gen coexprimiert, entstanden im Fall von MtSEO-F1 und MtSEO-F4 Proteinkomplexe, die Forisomenkörpern ähnelten, jedoch verschiedene Phänotypen aufwiesen. Für MtSEO-F2 und MtSEO-F3 konnte bei selbigem experimentellem Ansatz lediglich cytoplasmatisch lokalisiertes Protein detektiert werden. Darüber hinaus wurde eine Coexpression von MtSEO-F1/MtSEO-F1venus und MtSEO-F4/MtSEO-F4venus in Hefe durchgeführt, um aufzuzeigen, dass derartige künstliche Forisomenkörper in größeren Mengen herstellbar sind. Des Weiteren können auch große Mengen artifizieller Forisome durch ausschließliche Expression von MtSEO-F1 oder MtSEO-F4 hergestellt werden.

Die Proteinbiochemie hat in den letzten Jahrzehnten große Fortschritte gemacht, jedoch ist die Aufreinigung rekombinant hergestellter Proteine häufig immer noch eine große Herausforderung. Dies gilt beispielsweise für membranassoziierte oder für toxische Proteine. Gerade im Falle von Enzymen ist häufig zu beobachten, dass die Enzymmenge und/oder -aktivität nicht in einem wünschenswerten Bereich liegt, so dass die Kosten für Assays oder dergleichen aufgrund der benötigten Mengen unverhältnismäßig hoch sind. Die Expression rekombinanter Proteine wiederum kann als solche problematisch sein; so werden manche dieser Proteine im Expressionsorganismus nicht korrekt gefaltet oder als *inclusion bodies* in einer inaktiven Form innerhalb der Zelle abgeschieden. Eine weitere Anforderung an die Produktion ist die Wiederverwertbarkeit von Enzymen, die oft durch Immobilisierung an Trägermaterialien (Agarose, Nylon) realisiert wird. Diese Immobilisierung bewirkt oft eine starke Reduktion der Enzymaktivität, sodass die Kosten für die nachfolgenden Assays unverhältnismäßig hoch sind. Auch die Aufreinigung insbesondere polyklonaler Antikörper, die in der Regel mittels chromatographischer Methoden erfolgt, ist nach wie vor verbesserungsbedürftig. Die Erfinder haben sich daher die Aufgabe gestellt, hier Abhilfe zu schaffen und Proteine bereitzustellen, die sich einerseits mit vertretbarem Aufwand herstellen lassen und die andererseits eine Struktur oder Form besitzen, die den Einsatz dieser Proteine für die vorstehend genannten Zwecke erleichtert bzw.die Ergebnisse bei ihrer Anwendung gegenüber den Ergebnissen bei Verwendung der bekannten, bisher hierfür genutzten Proteine oder sonstigen Materialien verbessert.

In Lösung dieser Aufgabe schlägt die Erfindung vor, artifizielle Forisomenkörper zur Enzymimmobilisierung oder zur Aufreinigung rekombinanter Proteine zu verwenden. Diese können an vielen Stellen in der Proteinchemie Verbesserungen und Vereinfachungen herbeiführen, und zwar mittels darin in Form von Fusionsproteinen enthaltenen biochemisch aktiven Strukturen. Werden über die Fusion enzymatische Funktionen in die Forisome eingeführt, so können die Forisome als Träger dienen, an die die Enzyme ortsfest gekoppelt sind, so dass die Anbindung an eine Fremdmatrix überflüssig wird. Die Forisome können aber auch eine Schutzfunktion für das angekoppelte Fremdprotein besitzen, z.B. in Hinblick auf die Herstellung rekombinanter Proteine, deren Aufreinigung erleichtert wird: Das Fremdprotein kann in Form der Forisome leicht isoliert und anschließend bei Bedarf mit Hilfe geeigneter Proteaseschnittstellen und korrespondierender proteolytischer Enzyme aus diesen herausgelöst werden. Werden über die Fusion antigene Strukturen in die Forisome eingeführt, können diese als solche bei der Aufreinigung von Antikörpern genutzt werden. Zusätzlich können die erfindungsgemäßen Körper durch gezielte Variation ihrer Adhäsionseigenschaften oder Konformationsänderungs-Eigenschaften Aufgaben in der Mikromechanik übernehmen.

Aus den bisherigen, oben zitierten Arbeiten im Zusammenhang mit der Analyse der SEO-F-Gene und -Proteine ist bekannt, dass ein Fusionsprotein aus einem SEO-F1- oder SEO-F4-Protein mit einem fluoreszierenden Tag zusammen mit den entsprechenden nativen Proteinen Forisomenkörper bildet. Die Erfinder der vorliegenden Erfindung mussten jedoch feststellen, dass die Herstellung von Forisomen aus oder mit Fusionsproteinen beliebiger SEO-F-Einheiten mit beliebigen Proteinen nicht möglich ist. Es ist ihnen dennoch gelungen, artifizielle Forisomenkörper mit Fremdproteinen zu erzeugen, die sich für die erfindungsgemäßen Verwendungszwecke eignen. Diese Forisomenkörper lassen sich in Hefen exprimieren, weshalb große Mengen davon zur Verfügung gestellt werden können. Es hat sich nämlich gezeigt, dass sich SEO-F-Proteine bzw. Teilstücke davon sowohl C- also auch N-terminal mit einer Vielzahl von Proteinen, ggf. auch Peptiden kombinieren lassen und dabei Forisomenkörper bilden, sofern eine der nachstehenden Bedingungen erfüllt ist.

Demnach wird die Aufgabe der Erfindung durch die Verwendung eines artifiziellen Forisomenkörpers zur Enzymimmobilisierung oder zur Aufreiningung rekombinanter Proteine gelöst, wobei der artifizielle Forisomenkörper ein Fusionsprotein aus mindestens einem SEO-F-Protein oder einem mindestens 50 Aminosäuren umfassenden Teilstück davon und mindestens einem weiteren Protein oder Peptid umfasst, mit Ausnahme von Forisomenkörpern, die ausschließlich oder teilweise aus einem fluoreszierenden Fusionsprotein aufgebaut sind, wobei
(a) das weitere Protein oder Peptid eine Masse von maximal 30 kDa, vorzugsweise von maximal 25 kDa, aufweist und der Forisomenkörper kein unfusioniertes SEO-F-Protein oder eine C-terminal um bis zu 45 Aminosäuren und/oder N-terminal um bis zu 13 Aminosäuren deletierte Form dieses Proteins umfasst, oder
(b) der Forisomenkörper weiterhin ein unfusioniertes SEO-F-Protein oder eine C-terminal um bis zu 45 Aminosäuren und vorzugsweise bis zu 43 Aminosäuren und/oder N-terminal um bis zu 13 Aminosäuren deletierte Form dieses Proteins umfasst, wobei das unfusionierte SEO-F-Protein aus solchen Proteinen ausgewählt ist, die die Eigenschaft besitzen, in Abwesenheit weiterer SEO-F-Proteine homomere Forisomenkörper ausbilden zu können, oder
(c) das weitere Protein oder Peptid ein Teilstück eines zweiten, vom ersten SEO-F-Protein verschiedenen SEO-F-Proteins ist, mit der Maßgabe, dass eines der beiden SEO-F-Proteine in unfusionierter Form in der Lage ist, homomere Forisomenkörper auszubilden, und das Fusionsprotein aus einem N-terminalen SEO-F-Protein-Teilstück sowie einem C-terminalen SEO-F-Protein-Teilstück besteht, wobei die Teilstücke ein SEO-F-Protein darstellen, das vollständig ist oder das C-terminal um bis zu 50 Aminosäuren und/oder N-terminal um bis zu 13 Aminosäuren deletiert ist, mit der Maßgabe, dass ggf. eine beliebige Anzahl der vier Cysteine, die in den nativen SEO-F-Proteinen im C-terminalen Abschnitt zwischen AS 600 und AS 670 vorhanden sind, gegen Aminosäuren ausgetauscht sein können, die keine S-S-Brückenbindung ausbilden können.

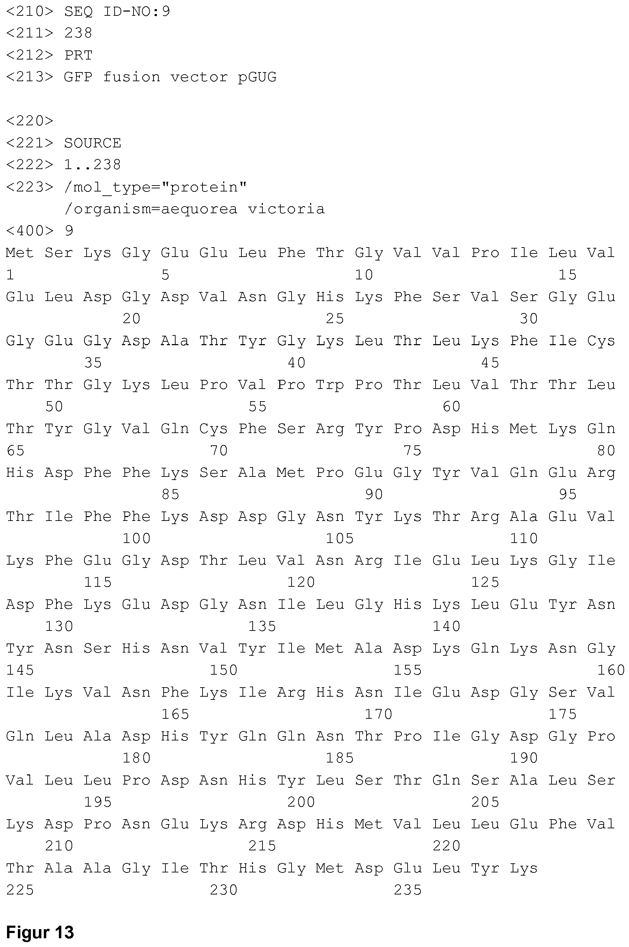

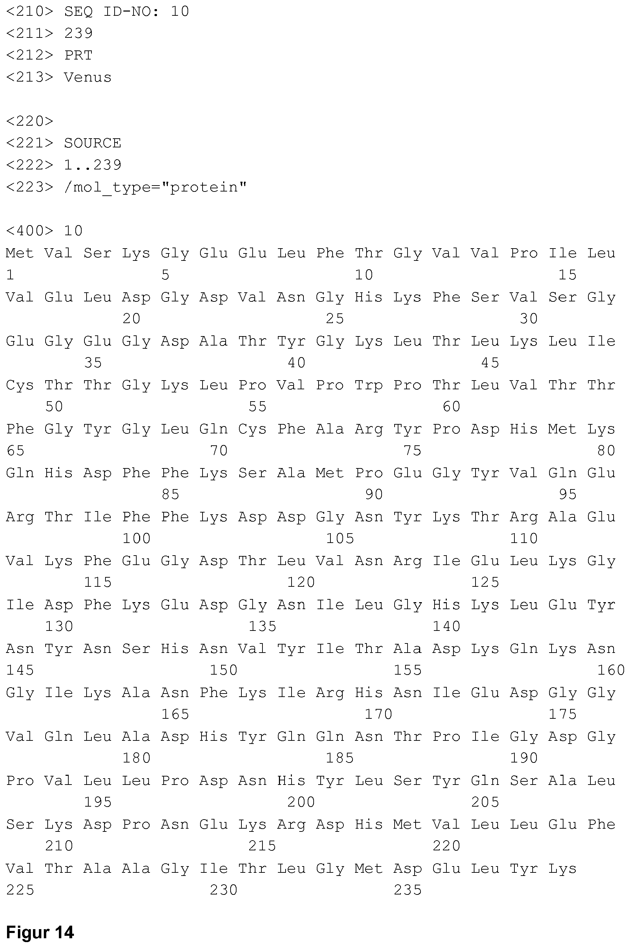

Wie bereits oben erwähnt, sind Forisomenkörper aus den Kombinationen der Fusionsproteine MtSEO-F1venus und MtSEO-F4venus mit entsprechenden nativen Proteinen wie MtSEO-F1/MtSEO-F1venus und MtSEO-F4/MtSEO-F4venus aus dem Stand der Technik bekannt. Diese sindvom Schutzbereich des Patentes ausgenommen. Sie wurden zum Zweck der Detektion von Forisomen hergestellt, während die vorliegende Erfindung in keiner Weise hierauf, sondern auf die Lösung spezifischer, in der Proteinchemie auftretender Probleme zielt. Deshalb fallen auch alle solchen Forisomenkörper nicht in den Schutzbereich des Patentes, die aus oder mit Fusionsproteinen hergestellt sind, in denen ein SEO-F-Protein oder -Peptid mit dem GFP-Protein (siehe **Figur 13** sowie SEQ ID NO: 9) oder dem Venusprotein (siehe **Figur 14** sowie SEQ ID NO: 10) oder einem Teilstück oder einer (artifiziellen) Abwandlung davon fusioniert ist, sofern das Fusionsprotein fluoreszierend ist, gegebenenfalls unter Anregungslicht (z.B. blauem oder ultraviolettem Licht). Auch Forisomenkörper, die einen Aufbau unter Einschluss von Fusionsproteinen mit anderen fluoreszierenden oder auf anderem Wege optisch detektierbaren Peptiden und Proteinen wie z.B. chemilumineszierenden Proteinen besitzen, sollen von der vorliegenden Erfindung nicht umfasst sein, zumindest sofern diese im nachstehend definierten Sinne nicht biochemisch aktiv oder aktivierbar sind. Im weitesten Sinne kann dies gegebenenfalls für alle solchen Forisomenkörper aus oder mit Fusionsproteinen gelten, deren Proteinanteil, der nicht aus einem SEO-F-Molekül stammt, keinen anderen Zweck erfüllen kann oder in der Regel erfüllen soll, als den Nachweis des Vorliegens der gesuchten Fusion. Die genannten Ausnahmen erstrecken sich selbstverständlich auch auf alle erfindungsgemäßen Produkte, die die genannten Forisomenkörper enthalten oder mit denen sich diese bzw. die entsprechenden Fusionsproteine herstellen lassen.

Ferner ist hierin die Herstellung von Forisomen aus Fusionsproteinen beschrieben, die den Forisomen eine artifizielle biochemische Aktivität oder Aktivierbarkeit oder veränderte mechanische Eigenschaften verleihen. Daher wird das weitere Protein oder Peptid ausgewählt unter biochemisch aktiven oder aktivierbaren Proteinen oder Peptiden sowie Teilstücken eines zweiten SEO-F-Proteins.

Erfindungsgemäß umfasst der Ausdruck "biochemisch aktive oder aktivierbare Proteine oder Peptide" unter anderem alle Proteine, die dem Stoffumsatz dienen wie insbesondere - aufgrund ihrer biokatalytischen Wirkung - Enzyme, alle Proteine, die eine Immunreaktion auslösen können oder therapeutischen Nutzen haben, wie insbesondere Antikörper und Antigene, alle Peptide oder Proteine, die Bindungsstellen für Fremdproteine oder -peptide besitzen, sowie sonstige biotechnologisch nutzbare Proteine und Peptide. Als "biotechnologisch nutzbar" sind erfindungsgemäß z.B. alle solchen Proteine und Peptide anzusehen, deren Synthese für medizinische Anwendungen oder diagnostische Methoden von Bedeutung sein können. Viele Proteine oder Peptide lassen sich aufgrund einer Affinitätsreaktion mit einem substratgebundenen biologischen oder biochemisch erzeugten Material auf einem Substrat immobilisieren, um eine Wiederverwertbarkeit zu erreichen. Auch diese fallen erfindungsgemäß unter den Begriff "biotechnologisch nutzbar". Nicht umfasst von dem Ausdruck sind dagegen Proteine oder Peptide, die (ausschließlich) dem Nachweis der Bildung des Fusionsproteins dienen, wie optisch detektierbare, insbesondere fluoreszierende Proteine, vor allem, wenn diesen eine biokatalytische Wirkung oder eine weitere der obengenannten Eigenschaften abgeht.

Die Erfinder konnten feststellen, dass sich Forisomenkörper in Hefen in der Regel immer dann bilden lassen, wenn die erfindungsgemäßen Fusionsproteine mit einem unfusionierten, beispielsweise nativen SEO-F-Protein coexprimiert werden, sofern dieses SEO-F-Protein die Eigenschaft besitzt, in Abwesenheit weiterer SEO-F-Proteine homomere Forisomenkörper auszubilden, siehe Bedingung (b). Dies dürfte der Tatsache geschuldet sein, dass wegen der Anwesenheit des Homomere bildenden SEO-F die Zahl und Ausprägung der für die Assemblierung relevanten Strukturen relativ hoch ist.

Überraschend konnten die Erfinder jedoch feststellen, dass sich die oben definierten Fusionsproteine in Hefen auch ohne die Gegenwart solcher unfusionierter SEO-F-Proteine zu Forisomenkörpern zusammenlagern, wenn der Anteil an Fremdprotein eine gewisse Größe nicht überschreitet. Die Erfinder stellten fest, dass hierfür der Nicht-SEO-F-Teil eine Masse von höchstens 30 kDa besitzen sollte. Vorteilhafter ist es, diese Grenze bereits bei ca. 25 kDa zu ziehen (Bedingung (a)). Die so erhältlichen Forisomenkörper sind etwas dünner und faseriger, lassen sich aber dennoch aufreinigen.

Zur Bedingung (c) der Definition der artifiziellen Forisome ist zu erwähnen, dass erfindungsgemäß festgestellt werden konnte, dass auf artifizielle Weise SEO-F-Fusionsprodukte hergestellt werden können, die jeweils alle für ein SEO-F-Protein erforderlichen Eigenschaften besitzen. Vorraussetzung hierfür ist, dass wenigstens ein Teil des Fusionsproteins aus einem SEO-F-Protein stammt, das homomere Forisomen bilden kann. Es wird vermutet, dass in diesen Proteinen die Strukturen stärker ausgeprägt sind, die zur Assemblierung und damit Forisomenbildung beitragen. Die vorgenannten Möglichkeiten einer gewissen Deletion, die im C-terminalen Bereich weiter getrieben werden kann als im N-terminalen Bereich, ist nach vorläufiger Auffassung der Erfinder der Tatsache geschuldet, dass sich die für die Assemblierung relevanten Strukturen nicht in diesen Bereichen befinden. Aufgrund der von den Erfindern aufgefundenen Möglichkeit, unter den Bedingungen gemäß (c) zu artifiziellen SEO-F-Proteinen zu gelangen, die sich gegebenenfalls homomer, d.h. ohne die Anwesenheit eines anderen, beispielsweise unfusionierten Proteins, zu Forisomen zusammenlagern, wird die Herstellung von Forisomen eröffnet, deren Assemblierungsvermögen in geeigneter Weise gesteuert, z.B. erhöht werden kann. Auf diesem Wege können die mechanischen Eigenschaften solcher Forisome an die gewünschten Anwendungen angepasst werden. Beispielsweise können die Bedingungen (Ca²⁺-Gehalt und/oder pH-Wert und/oder elektrische Stimuli) für die Konformationsänderungen variiert werden, so dass die Forisomen auch unter Bedingungen technisch verwendet werden können, die bei nativen Forisomen keine Konformationsänderung hervorrufen können.

Erfindungsgemäß kann das Fusionsprotein das weitere Protein C-terminal, d.h. bezogen auf den Klonierungsvektor und die Ablesung der DNA "upstream", oder N-terminal, d.h. bezogen auf den Klonierungsvektor und die Ablesung der DNA "downstream" enthalten.

Besondere Vorteile lassen sich mit der vorliegenden Erfindung auf den folgenden Gebieten erzielen:
a) Enzymimmobilisierungen werden bei industriell gebräuchlichen Enzymen genutzt, da sie den Vorteil der Wiederverwertbarkeit der Enzyme und eine Minimierung von Verunreinigungen des Enzymprodukts bieten. Allerdings wird die Enzymstabilität und -Aktivität im Vergleich zur löslichen Enzymform in der Regel durch das verwendete Trägermaterial verringert. Bisher erfolgen Enzymimmobilisierungen meist durch Adsorption, Einschluss, Vernetzung oder kovalente Bindung des Enzyms an Trägermaterialien. Nachteile der Immobilisierungsmethoden sind z.B. eine unzureichende Befestigung des Enzyms bei Adsorption und Einschluss, der Einsatz toxischer Chemikalien bei der Vernetzung und die Blockierung essentieller funktioneller Aminosäuregruppen bei der Einführung kovalenter Bindungen. Als Trägermaterialien werden bisher synthetische Polymere wie Acrylatharze, Hydrogele und Kieselerde, smarte Polymere wie PNIPAM oder Biopolymere wie Agarose, Zellulose, Stärke und Chitosan eingesetzt. So wird an Agarose-Kügelchen immobilisierte Glukose-6-Phosphatdehydrogenase mit einer Aktivität von 1000-1750 Units/ Gramm Agarose kommerziell angeboten. Die Aufreinigung des Enzyms und die anschließende Kopplung des Enzyms an das Trägermaterial stellen dabei zwei getrennte Arbeitsschritte dar, wobei die Enzymaktivität nach der Immobilisierung stark verringert wird.
b) Die Expression rekombinanter Proteine kann, je nach Eigenschaften des Proteins, problematisch sein. Beispielsweise beeinflussen toxische Proteine die Vitalität des Expressionsorganismus und verringern die Produktionsmenge des rekombinanten Proteins. Andere Proteine werden nicht korrekt gefaltet oder als *inclusion bodies* in einer inaktiven Form innerhalb der Zelle abgeschieden. Weitere Probleme können bei der Aufreinigung des rekombinanten Proteins auftreten. So wird z.B. die Isolierung von Membranproteinen durch die Interaktion mit Membranbestandteilen erschwert oder Proteine werden während des Aufreinigungsprozesses degradiert. Des Weiteren ist der Prozess der Proteinaufreinigung in der Regel sehr kostenintensiv und kommt häufig nicht ohne die Verwendung größerer Mengen umweltschädlicher Chemikalien aus. In der Praxis unterteilt sich die Aufreinigung auch bei industriellen Herstellungsverfahren in mehrere Schritte. Die Arbeitsschritte umfassen dabei Ausfällungen, Filtrationen oder chromatographische Methoden. Die wichtigsten Kriterien bei diesen Methoden sind die Aufreinigungseffizienz, die Kosteneffizienz und deren biologische Nachhaltigkeit. Ausfällungen sind z.B. sehr kostengünstig, führen aber nur zu einem geringen Reinheitsgrad und setzen den Einsatz großer Mengen von Chemikalien voraus, während Filtrationen oder chromatographische Methoden oft sehr kostenintensiv sind. Aus diesem Grund ist die Entwicklung neuartiger Aufreinigungsmethoden, die die Produktreinheit steigern, die Kosten reduzieren und den Einsatz von Chemikalien minimieren, von gesteigertem industriellem Interesse.
c) Die Gewinnung von polyklonalen Antikörpern erfolgt durch die Injektion der jeweiligen Antigene (Proteine oder Peptide) in Tiere. Nach einigen Wochen kann das polyklonale Serum aus dem Blutkreislauf des Tieres entnommen werden. Für die Gewinnung monoklonaler Antikörper werden Plasma-Zellen aus Milz oder Lymphknoten immunisierter Tiere isoliert, mit einer Tumorzelle verschmolzen und in Sterilkultur kultiviert. Nach mehrfachen Vereinzelungen können Hybridoma-Kulturen gewonnen werden, die aus einer einzelnen Zelle hervorgehen und den gewünschten monoklonalen Antikörper sezernieren. Insbesondere bei polyklonalen Antikörpern, seltener bei monoklonalen Antikörpern, sind im Serum neben den gewünschten Antikörpern auch ungewünschte Antikörper (z.B. Keratin-Antikörper) und/oder mit der Detektion interferierende Substanzen (z.B. Proteine, die dem eingesetzten Antigen ähneln, oder Proteine, die aggregieren und Nachweismethoden stören) enthalten. Diese Substanzen müssen von dem gewünschten Antikörper abgetrennt werden. Dies wird bisher durch chromatographische Methoden bewerkstelligt. Dazu wird das Antigen an eine Säulenmatrix gebunden. Danach wird die Matrix mit den "unreinen" Antikörper-Lösungen inkubiert, so dass die spezifischen Antikörper an das Antigen und somit an die Matrix binden können. Nachdem die Matrix gewaschen wurde, werden die Antikörper von der Säule eluiert (z.B. durch eine Lösung mit saurem pH-Wert). Eine Vereinfachung dieses mühsamen Verfahrens und eine Verbesserung der Effektivität ist außerordentlich wünschenswert.
d) Die Vorteile der Erfindung erstrecken sich jedoch nicht nur auf die Herstellung und die Eigenschaften von Fremdproteinen; auch auf dem Gebiet der Forisomen selbst kann sie vorteilhaft eingesetzt werden: Wie oben erwähnt, sind Forisome pflanzliche Mechanoproteine, die aufgrund ihrer durch Calcium- oder pH-abhängigen Konformationsänderung z.B. als Steuermodule in mikrofluiden Systemen eingesetzt werden können. So konnten A. Q. Shen et al. in Smart Struct. Syst. 2, 225-235 (2006) und K. Uhlig et al. in J. Microelektromech. Sys. 17, 1322-1328 (2008) zeigen, dass sich der Fluss fluoreszierender Partikel in Mikrokanälen durch darin integrierte Forisome steuern lässt. Eine gezielte, permanente Befestigung der Forisome ist bisher jedoch nur manuell mit Hilfe von Mikromanipulationstechniken und unter einem sehr hohen Zeit- und Arbeitsaufwand möglich. So wurde von Shen *et al.* und Uhlig *et al.* (a.a.O.) die natürliche Glasadhäsion ausgenutzt. Forisome haften dabei an Oberflächen, wenn sie an diese gedrückt werden. Jedoch gewährt die Adhäsion keine permanente Befestigung des Forisoms im Flüssigkeitsstrom. Ausserdem verringert sich die Reaktionsstärke der Forisome, wenn sie an Oberflächen adherieren (G. A. Noll et al., Bioeng. Bugs 2, 111-114 (2011)).

Auch die Bereitstellung von Forisomen, deren Konformationsänderungs-Eigenschaften unter der Einwirkung der bekannten Stimuli verändert ist (z.B. mit einem Übergang vom kondensierten in den dispersen Zustand und vice versa bei einem anderen pH-Wert oder einer andere Ca²⁺-Konzentration) wäre wünschenswert.

Den Erfindern der vorliegenden Erfindung ist es gelungen, ein Material bereitzustellen, mit dem sich Vorteile auf allen vier der genannten Gebiete erzielen lassen. Dabei wurde festgestellt, dass die Expression von Fusionsproteinen häufig dann möglich ist, wenn eine Co-Expression eines Forisomen-Proteins, das unabhängig vom Vorhandensein weiterer Forisomen-Untereinheiten Homomere bilden kann, in derselben Zelle erfolgt. Die Expression des Fusionsproteins allein führt dagegen nur bei Einsatz kleinerer Fremdproteine zu einem nutzbaren Produkt, während in den anderen Fällen keine Forisomenkörper gebildet werden und stattdessen das Protein in löslicher Form vorliegt oder als "*inclusion body*" in der Zelle abgelagert wird.

Erfolgt die Coexpression eines Fusionsproteins und einer homomere Forisomenkörper bildenden SEO-F-Untereinheit und/oder wird ein Fusionsprotein mit einer relativ kleinen Fremdprotein-Einheit exprimiert, so lassen sich in Pflanzen und in Hefen stabile Forisomenkörper exprimieren, die trotz des Vorhandenseins eines Fremdproteins oder -peptids im Wesentlichen die Form nativer Forisomen besitzen. Damit bietet die Erfindung die Möglichkeit der Verwendung individuell modulierbarer, funktionalisierter, artifizieller Forisomen. Nicht nur dies war überraschend, sondern insbesondere auch die Feststellung, dass die Assemblierung des Forisomenkörpers dabei die funktionelle Wirkung des Fremdproteins nicht behindert. Am Beispiel von mit Enzymen fusionierten SEO-F-Einheiten konnte gezeigt werden, dass der Forisomenkörper die Aktivität des Fremdproteins im Vergleich zu kommerziell verwendeten Immobilisierungsträgern weniger stark reduziert, was für alle Fusionsproteine angenommen werden darf, auch wenn sich dies bei einer Reihe von anderen Proteinen mangels quantitativer Vergleichsmöglichkeiten nicht nachweisen lässt.

Die Verwendung von MtSEO-F1 und MtSEO-F4 ist besonders bevorzugt; es lassen sich jedoch genauso SEO-F-Untereinheiten aus anderer Quelle einsetzen.

Es konnte festgestellt werden, dass es nicht erforderlich ist, dass die gesamte Kette der jeweiligen nativen SEO-F-Untereinheit im Fusionsprotein vorhanden ist. Stattdessen genügt ein ggf. sogar relativ kleiner Teil davon, z.B. ein Bereich von ca. 60 bis 250 Aminosäuren Länge, wie die Erfinder mittels Fusionierung mit fluoreszierenden Proteinen feststellen konnten. Dies korrespondiert auch mit der Feststellung, dass das Vorhandensein von Homomere bildenden Proteinen wie SEO-F1 bzw. SEO-F4 bestimmt, ob Forisome ausgebildet werden, wenn das Fremdprotein eine gewisse Größe überschreitet.

Der SEO-F-Bestandteil des Fusionsproteins kann von allen SEO-F-Untereinheiten stammen; bevorzugt stammt er von den Untereinheiten SEO-F1, SEO-F2 und SEO-F4, insbesondere von MtSEO-F1, MtSEO-F2 und MtSEO-F4.

Das coexprimierte, unfusionierte SEO-F-Protein, sofern vorhanden, sollte im Wesentlichen oder zumindest in großen Teilen vollständig sein, um die Ausbildung der Forisomen zu gewährleisten. Die Erfinder haben jedoch festgestellt, dass nicht die gesamte Kette der jeweiligen Untereinheit vorliegen muss. Eine Deletion von bis zu 13 Aminosäuren N-terminal und/oder von bis zu 45 Aminosäuren C-terminal kann hingenommen werden, ohne dass die Verwendung der artifiziellen Forisomenkörper der vorliegenden Erfindung beeinträchtigt würde. Die Forisomenkörper nach der Verwendung der vorliegenden Erfindung können aus einer beliebigen Anzahl von Untereinheiten aufgebaut sein; in der Regel genügt bereits eine Spezies einer nicht fusionierten SEO-F-Untereinheit in Kombination mit einer Spezies eines Fusionsproteins oder eine Spezies eines Fusionsproteins alleine, wenn dessen Fremdproteinanteil die genannte Größe nicht überschreitet. Die Forisomenkörper bestehen in der Regel aus ungefähr 10⁶-10⁷ einzelnen Proteinketten, wobei ggf. das Verhältnis der Anzahl der nicht fusionierten SEO-F-Untereinheiten zur Anzahl der Fusionsproteine bei etwa 4:1 - 1:1 liegt, je nach Art und Größe des Fremdproteins.

Einzelne Forisomenkörper nach der Verwendung der Erfindung weisen in der Regel nur eine Art von Fusionsprotein auf; sie können aber auch mehrere unterschiedliche Fusionsproteine enthalten; ein spezifisches, besonders günstiges Beispiel hierfür wird nachstehend unter Punkt 1) erläutert.

Die Herkunft der jeweiligen Forisomen-Untereinheiten aus nativen Quellen spielt für die Erfindung keine Rolle. Erfindungsgemäße Forisomenkörper konnten mit SEO-F-Genen beispielsweise aus den Organismen *Dipteryx panamensis, Pisum sativum, Vicia faba, Canavalia gladiata* und *Lotus japonicus* hergestellt werden. Daraus lässt sich schließen, dass die entsprechenden Gene aller Pflanzen der Fabacea-Familie erfindungsgemäß eingesetzt werden können. Darüber hinaus können auch genetisch veränderte oder synthetische SEO-F-Gene bzw Forisomen-Untereinheiten verwendet werden, zumindest sofern die für alle Gene dieser Pflanzenfamilie konservierten Bereiche erhalten bleiben bzw. vorhanden sind.

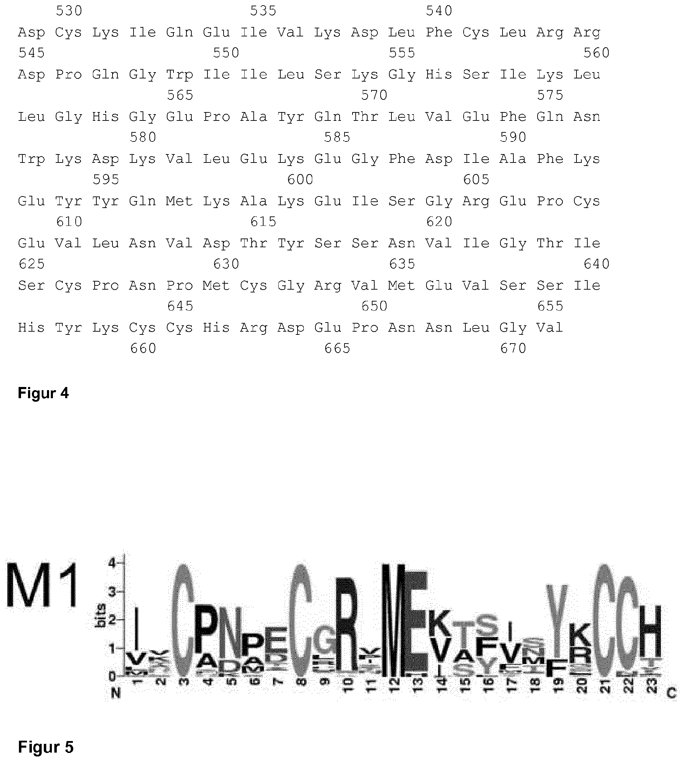

Es wird seit längerem vermutet, dass eine Abfolge von vier Cysteinen in der Aminosäuresequenz der verschiedenen Forisomen-Untereinheiten auf deren Struktur und Stabilität einen großen Einfluss hat. Diese Cysteine befinden sich in allen drei Forisomen-Untereinheiten SEO-F1, SEO-F2 und SEO-F4 im C-terminalen Abschnitt der Aminosäuresequenz (nach Position 600), und zwar jeweils in einem stark konservierten Motiv CPNP**X**CGRVMEV**X**S**XX**YKCC (X steht für eine variable Aminosäure). Dieses Motiv ist in allen SEO-Genen (d.h. auch in denen anderer Pflanzenfamilien) hoch konserviert. Das entsprechende Sequenz-Logo ist unter **Figur 5** gezeigt. Die Erfinder konnten jedoch zeigen, dass das Vorhandensein dieses Abschnitts nicht wesentlich für die Ausbildung von Forisomen ist. Denn, wie oben erwähnt, ist es möglich, als coexprimiertes unfusioniertes SEO-F oder als aus zwei SEO-F-Komponenten bestehendes Fusionsprotein ein SEO-F-Protein einzusetzen, das C-terminal um bis zu 45 oder gar 50 Aminosäuren verkürzt ist, ohne dass die Eigenschaft der Zusammenlagerung der Proteinketten nach der erfindungsgemäßen Verwendung verloren ginge. Wenn jedoch ein SEO-F1 oder SEO-F4 in voller Länge oder zumindest mit einer Sequenzlänge, bei der mindestens ein Teil oder sogar das gesamte genannte konservierte Motiv vorhanden ist, eingesetzt wird, besitzen die genannten Cysteine offensichtlich eine nicht unerhebliche Rolle. Es hat sich nämlich herausgestellt, dass sich dann, wenn die genannten Cysteine, z.B. mittels "site directed mutagenesis", teilweise oder ganz durch Aminosäuren ersetzt werden, die keine Disulfid-Brückenbildung ermöglichen, z.B. durch Glycin oder Alanin, die Konformations-Zustände der Forisomenkörper verändern wie folgt: Werden die letzten beiden der genannten Cysteine (Cysteine C21 und C22 im Sequenz-Logo) mutiert, lagern sich die Proteinfibrillen nicht mehr in allen Fällen zum Forisomenkörper zusammen, sondern können ein ungeordnetes Fasernetzwerk bilden. Daher wird vermutet, dass die Disulfidbrücken zwischen diesen Cysteinen zweier SEO-F-Untereinheiten für die geordnete Zusammenlagerung der Proteinfibrillen verantwortlich sind. Wird dagegen mindestens eines der ersten beiden dieser Cysteine (Cysteine C3 und C8 im Sequenz-Logo) mutiert, bildet sich nach deren Expression ein typischer Forisomenkörper aus, der sich jedoch nach Zugabe von Calciumionen und NaHSO₃ vollständig auflöst. Calcium löst dabei die Abstoßung der Proteinfibrillen aus, während die Zugabe von NaHSO₃ noch bestehende Disulfidbrücken auflöst. Es ist daher anzunehmen, dass die Cysteine C3 und C8 an der Verknüpfung einzelner Untereinheiten zu den Fasern beteiligt sind, weshalb das Protein nach dessen Mutation in seine lösliche Form überführt werden kann.

Die faserförmigen Körper nach der erfindungsgemäßen Verwendung können vorteilhafte Eigenschaften besitzen. Der Ausdruck "artifizielle Forisome", wie er hierin verwendet wird, umfasst die genannten Fasernetzwerke mit.

Besonders günstig ist die Herstellung löslicher Forisomen-Körper wie oben beschrieben, denn hierdurch kann die Aufreinigung von Proteinen erleichtert sein, wie die nachstehenden Beispiele zeigen.

Die Herstellung der Forisomenkörper erfolgt, wie bereits voranstehend erwähnt, vorzugsweise in Pflanzen- oder Hefezellen, wobei die Verwendung von Hefezellen besonders günstig ist, weil sie die Herstellung großer Mengen an artifiziellen Forisomenkörpern ermöglicht. Die erfindungsgemäße Verwendung ist daher auch auf entsprechend transformierte Zellen gerichtet. Und ferner sind hierin neue Vektor-Konstrukte beschrieben, mit deren Hilfe die Forisomenkörper für die erfindungsgemäße Verwendung erzeugt werden können.

Nachstehend soll die Erfindung anhand mehrerer Beispiele näher erläutert werden, die einerseits die Breite der Anwendungsmöglichkeit der erfindungsgemäßen Verwendung demonstrieren, andererseits die einzelnen Maßnahmen konkretisieren, anhand derer es dem Fachmann möglich ist, die Erfindung auszuführen.

### 1) Forisomenkörper mit enzymgekoppelten Fusionsproteinen

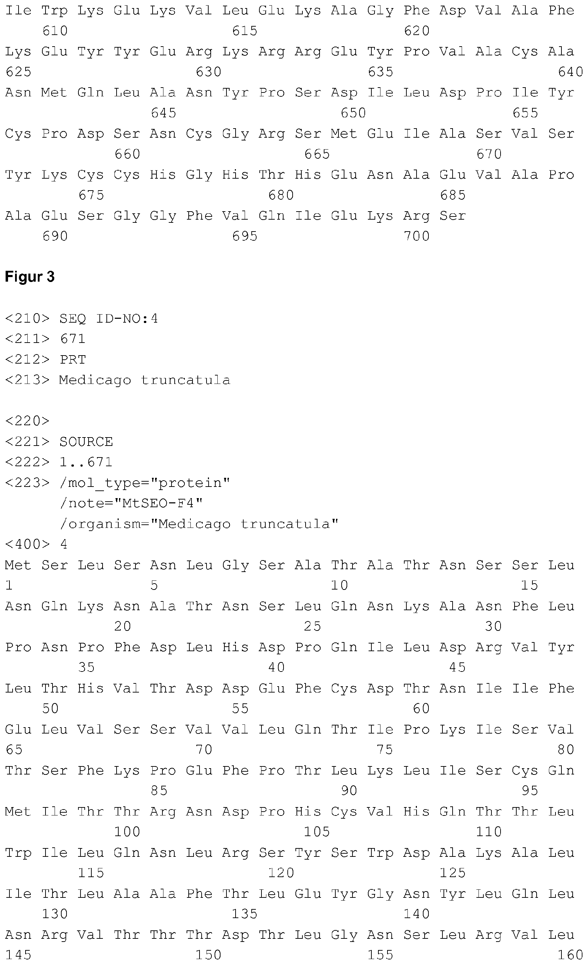

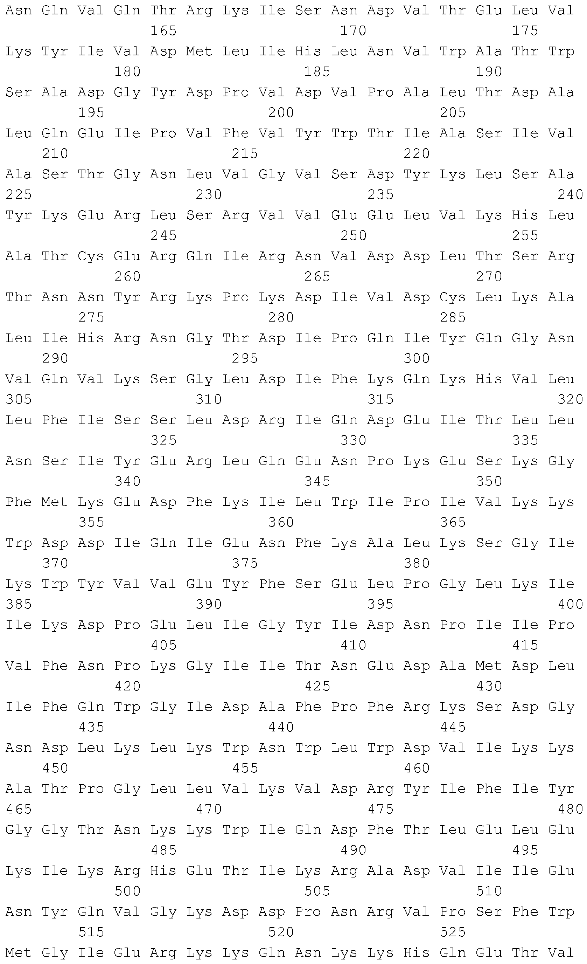

Durch die Kopplung von Enzymen an SEO-F-Proteine können artifizielle Forisome dahingehend funktionalisiert werden, dass sie als Trägermaterial für Enzyme dienen. Enzyme können somit immobilisiert werden. Enzymgekoppelte Forisome sind dabei wie folgt aufgebaut: Sie bestehen aus einer ersten, auch ggf. verkürzten SEO-F-Untereinheit, die mit einem Enzym fusioniert ist, sowie ggf. einer zweiten SEO-F-Einheit, ausgewählt unter SEO-F1 und SEO-F4, die bei Bedarf bzw. auf Wunsch wie oben angegeben deletiert sein kann. Das Fusionsprotein kann das fusionierte Enzym entweder C- oder N-terminal tragen. Herstellen lassen sie sich durch Coexpression in geeigneten Expressionsorganismen wie Hefen (z.B. *Saccharomyces cerevisiae*), Bakterien (z.B. *Escherichia coli*) oder Pflanzen (z.B. Tabak). Die (Co-)Expression in Hefen ist besonders bevorzugt. Die entstehenden enzymgekoppelten Forisome zeichnen sich durch eine hohe Stabilität aus. Sie werden aus dem Expressionsorganismus isoliert (z.B. durch einen Aufschluss der Hefezellen) und z.B. durch Zentrifugation/Dichtegradientenzentrifugation von Zellbestandteilen abgetrennt. Um zu überprüfen, ob das gekoppelte Enzym aktiv ist, dienen entsprechende Enzymaktivitätstests. Mit Glukose-6-Phosphat-Dehydrogenase als an den N-Terminus der Forisomen-Untereinheit fusioniertem Enzym konnte eine im Vergleich zu kommerziell erhältlichem immobilisiertem Enzym deutlich höhere Enzymaktivität gemessen werden (2700 Einheiten/ Gramm Forisom im Vergleich zu 1000-1750 U/ g Agarose, siehe Figur 4). Das Enzym konnte dabei direkt in immobilisierter Form aus dem Produktionsorganismus isoliert werden, was die Enzymherstellung um den Schritt der Trägermaterialkopplung verringert. Dies stellt nicht nur eine Arbeitserleichterung dar, sondern bewirkt offensichtlich und überraschend eine extreme Aktivitätssteigerung. Mit Hexokinase und mit Phosphoglucoisomerase in vergleichbarer Weise hergestellte Fusionsproteine lieferten ähnliche Ergebnisse.

Erfolgt die Koexpression nicht nur mit einem, sondern mit zwei oder noch mehr Fusionsproteinen, wobei die Fusionspartner so ausgewählt sind, dass das Umsetzungsprodukt des ersten Enzyms als Substrat des zweiten Enzyms dient und dessen Umsetzungsprodukt ggf. als Substrat eines dritten Proteins usw., lassen sich Reaktionskomplexe generieren, an denen bestimmte Reaktionsketten ablaufen.

Im Fusionsprotein kann das Enzym auch am C-Terminus des SEO-F-Proteins gebunden vorliegen.

### 2) Aufreinigung rekombinanter Proteine

Wie oben erwähnt, lassen sich die artifiziellen Forisomenkörper nach der erfindungsgemäßen Verwendung auch als Aufreinigungssysteme für rekombinante Proteine nutzen. Diese werden dabei an eine SEO-F-Untereinheit fusioniert, und das Fusionsprotein wird ggf. mit einer zweiten SEO-F-Untereinheit coexprimiert, die in der Lage ist, in Abwesenheit anderer Untereinheiten Homomere zu bilden, wie voranstehend ausgeführt, beispielsweise in Hefe- oder Pflanzenzellen. Das rekombinante Protein kann im Fusionsprotein C-terminal oder N-terminal vorliegen und bei Bedarf mit einer Protease-Restriktionsschnittstelle versehen werden, um im Anschluss an die Aufreinigung eine Abspaltung des Fremdproteins vom Forisomenkörper zu gewährleisten. Die Isolierung und Aufreinigung der erhaltenen, artifiziellen Forisomen erfolgt anhand eines Zellaufschlusses und z.B. einer Zentrifugation/ Dichtegradientenzentrifugation.

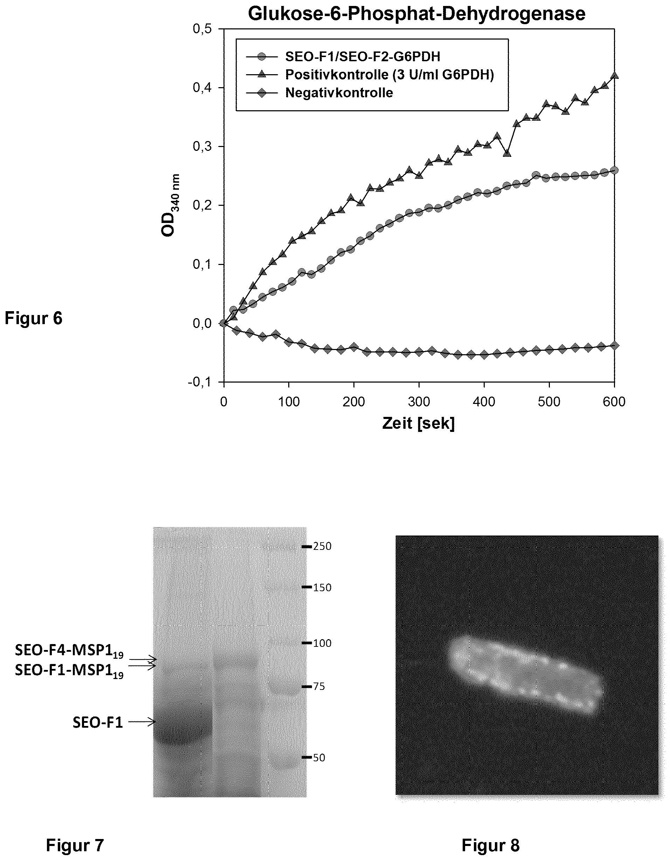

Alternativ kann das Proteinpolymer, insbesondere nach Mutation einer oder mehrerer der oben beschriebenen, C-terminalen, konservierten Cysteine, mittels hoher Ca²⁺-Konzentrationen (< 2 mM) oder mittels einer Kombination aus hoher Ca²⁺-Konzentration (< 2 mM) und reduzierenden Bedingungen (> 18,5 µM NaHSO₃) von dem festen Polymerzustand in einen löslichen Zustand überführt werden. Damit bietet die Forisomentechnologie ein vollkommen neues Aufreinigungssystem, das auf traditionelle Verfahren wie Ausfällung, Filtration und Chromatographie vollkommen verzichtet und stattdessen auf Zentrifugationsverfahren und dem Konformationszustand des Proteins beruht. Auf Basis dieser Technologie kann die Proteinaufreinigung einer Vielzahl von Proteinen vereinfacht und verbilligt werden. Zusätzlich bietet das Aufreinigungssystem den Vorteil, dass toxische Effekte oder Membraninteraktionen des aufzureinigenden Proteins durch die Forisomenkopplung minimiert bzw. unterbunden werden. So ließ sich beispielsweise das Malaria Antigen MSP1₁₉, dessen Aufreinigung aufgrund seiner starken Membraninteraktion auf anderem Wege äußerst schwierig ist, mit Hilfe der vorliegenden Erfindung erfolgreich aufreinigen. Dies lässt sich anhand der immunologischen Detektion des Antigens zeigen, die in Figur 7 dargestellt ist.

### 3) Aufreinigung von Antikörpern

Die Erfindung ermöglicht es, den Aufreinigungsprozess von polyklonalen oder monoklonalen Antikörpern mit artifiziellen Forisomen durchzuführen und damit die bisherigen chromatographischen Trennschritte zu vermeiden. Dazu wird das Antigen durch die zuvor beschriebenen Methoden *up-* oder *downstream* eines Forisomengens (MtSEO-F1, MtSEO-F2 oder MtSEO-F4 bzw. eines Teilstücks davon, wie oben definiert) kloniert. Die Antigen-MtSEO-F-Fusion wird anschliessend mit ggf. C- und/ oder N-terminal um bis zu 13 Aminosäuren deletiertem MtSEO-F1 und MtSEO-F4 in Hefe exprimiert. Dabei werden in den Hefen artifizielle Forisome gebildet, die das Antigen enthalten.

Die Hefen werden kultiviert, abzentrifugiert und aufgeschlossen. Die artifiziellen Forisome, die das Antigen tragen, liegen nun frei in der Lösung vor und können im Folgenden für die Aufreinigung polyklonaler oder monoklaner Antikörper genutzt werden.

Die artifiziellen Forisome mit Antigen werden mit Antikörperserum inkubiert. Dabei binden die spezifischen Antikörper an die artifiziellen Forisome. Die Forisome werden abzentrifugiert, gewaschen, und anschließend werden die Antikörper mittels einer pH-Verschiebung eluiert. Daraufhin wird die Antikörperlösung neutralisiert und kann nun für verschiedene Anwendungen genutzt werden (Western Blot, Immunopräzipitation ELISA, Antikörpertherapie, etc.).

### 4) Modifikation der Forisomen-Eigenschaften

Mit Hilfe der Erfindung können Forisomen artifiziell so modifiziert werden, dass sie neue, technologisch wertvolle Eigenschaften erhalten. Beispielsweise kann die Adhäsion von Forisomen an Oberflächen dadurch verbessert werden, dass sie mit Proteinen oder (Protein- oder Peptid-)Tags fusionierte SEO-F-Untereinheiten enthalten. Damit können sie in Mikrokanälen positioniert und immobilisiert werden. Beispiele hierfür sind die Fusion mit der B-Domäne aus dem *Staphylococcus aureus* Protein A, mit der Glutathion S-Transferase oder mit Biotin, mit denen sich eine gezielte Oberflächenfunktionalisierung der im Produktionsorganismus produzierten und anschließend isolierten artifiziellen Forisome erzielen lässt, derart, dass diese über kovalente Bindungen an mit IgG, mit Glutathion oder mit Streptavidin beschichtete Oberflächen angebunden werden können. Wiederum lassen sich stabile Forisome dann erhalten, wenn eine Coexpression einer SEO-F-Untereinheit, die in der Lage ist, in Abwesenheit anderer Untereinheiten Homomere zu bilden, mit dem Fusionsprotein erfolgt, oder der Fremdproteinanteil im Expressionsprodukt nicht zu hoch ist. Damit lässt sich das Problem ausräumen, das mit einer korrekten Positionierung von als Mechanoproteinkörper genutzten Forisomen auf Oberflächen oder in Mikrokanälen verbunden ist. Wenn anstelle der Fusion mit einem Fremdprotein eine Fusion mit einem weiteren SEO-F-Protein durchgeführt wird, erhält man Mechanoproteinkörper, deren Konformationsänderungs-Eigenschaften unter dem Einfluss der Ca²⁺-Konzentration und des pH-Werts verändert sind.

Die nachstehenden konkreten Ausführungsbeispiele sollen das Verständnis der Erfindung weiter vertiefen.

### Beispiel 1 - Enzymimmobilisierungen an artifiziellen Forisomen (Enzymkopplung)

I. Die Forisomengene MtSEO-F1, MtSEO-F2 und MtSEO-F4 wurden mit und ohne translationalem Stopcodon auf Basis von *M*. *truncatula* cDNA mit folgenden Oligonukleotiden amplifiziert (die Restriktionsschnittstellen sind unterstrichen):
   MtSEO-F1 fw NcoI: 5'-AGA ACC ATG GGA TCA TTG TCC AAT GGA ACT AAA C-3'
   MtSEO-F1 rev XhoI mit Stop: 5'-AGA CTC GAG TCA TAT CTT GCC ATT CTG TGG AGC-3'
   MtSEO-F1 rev XhoI ohne Stop: 5'-AGA CTC GAG CAT ATC TTG CCA TTC TGT GGA GC-3'
   MtSEO-F2 fw NcoI: 5'-AGA ACC ATG GGA TCC ACT GCA TTG TCC TAT AAT G-3'
   MtSEO-F2 rev XhoI mit Stop: 5'-AGA CTC GAG TCA AAT GCA GCA ACT ATC TGG-3'
   MtSEO-F2 rev XhoI ohne Stop: 5'-AGA CTC GAG ATG CAG CAA CTA TCT GGA-3'
   MtSEO-F4 fw NcoI: 5'- AGA ACC ATG GGA TCC CTT TCC AAC TTA GGA AG-3'
   MtSEO-F4 rev XhoI mit Stop: 5'-AGA CTC GAG TCA AAC ACC AAG ATT GTT TGG-3'
   MtSEO-F4 rev XhoI ohne Stop: 5'- AGA CTC GAG ACA CCA AGA TTG TTT GGT TC-3'

   Die Amplifikate wurde mit den Restriktionsenzymen NcoI/XhoI restringiert und in die korrespondieren Schnittstellen des pENTR4™-Vektors (Invitrogen, Deutschland) kloniert. Auf diese Weise wurden pENTR4-MtSEO-F-Vektoren mit und ohne Stopcodon hergestellt.
II. Die Gene der Enzyme Hexokinase 2 (HXK2), Phosphoglucoisomerase (PGI) und Glucose-6-posphatdehydrogenase (G6PDH) aus *Saccharomyces cerevisiae* wurden auf Basis von cDNA mit folgenden Oligonukleotiden amplifiziert (Restrikitionsschnittstellen sind unterstrichen).
   G6PDH fw XhoI: 5'-AGA CTC GAG AAT GAG TGA AGG CCC CGT C-3'
   G6PDH rev XhoI: 5'- AGACTCGAGCTAATTATCCTTCGTATCTTC
   HXK2 fw XhoI: 5'-AGACTCGAGAATGGTTCATTTAGGTCCAAA
   HXK2 bw XhoI: 5'-AGACTCGAGTTAAGCACCGATGATACCA
   PGI XhoI fw: 5'-AGACTCGAGAATGTCCAATAACTCATTCAC
   PGI XhoI rev: 5'- AGACTCGAGATCACATCCATTCCTTGAATTG
   Invertase XhoI fw: 5'-AGACTCGAGAGCATCAATGACAAACGAAAC
   Invertase XhoI rev: 5'- AGACTCGAGCTATTTTACTTCCCTTACTTGG

   Die Amplifikate wurden mit XhoI restringiert und in die korrespondierende Schnittstelle der pENTR4-MtSEO-F-Vektoren ohne Stop kloniert (siehe a)I.). Auf diese Weise wurden die Vektoren pENTR4-MtSEO-F1-G6PDH, pENTR4-MtSEO-F2-G6PDH, pENTR4-MtSEO-F4-G6PDH, pENTR4-MtSEO-F1-HXK2, pENTR4-MtSEO-F2-HXK2, pENTR4-MtSEO-F4-HXK2, pENTR4-MtSEO-F1-PGI, pENTR4-MtSEO-F2-PGI und pENTR4-MtSEO-F4-PGI erhalten.
III. Die Vektoren pENTR4-MtSEO-F1 mit stop und pENTR4-MtSEO-F4 mit stop wurden in die Hefevektoren 425GPD-ccdB (Addgene, USA) rekombiniert. Die resultierenden Expressionskonstrukte 425GPD-MtSEO-F1 und 425GPD-MtSEO-F4 wurden in den Hefestamm InvSc1 (Invitrogen, Deutschland) transformiert. Zur Selektion wurde die Komplementierung der Leucin-Auxotrophie des Hefestammes genutzt. Die resultierenden Hefen produzieren artifizielle Forisome aus MtSEO-F1 oder MtSEO-F4, die als Basis für die Enzymkopplung verwendet wurden.
IV. Die oben genannten pENTR4-Vektoren mit MtSEO-F-Enzym-Fusionen (siehe 1.II.) wurden mit dem Hefevektor 424GPD-ccdB (Addgene, USA) rekombiniert. Die resultierenden Vektoren (424GPD-MtSEO-F1-G6PDH, 424GPD-MtSEO-F2-G6PDH, 424GPD-MtSEO-F4-G6PDH, 424GPD-MtSEO-F1-HXK2, 424GPD-MtSEO-F2-HXK2, 424GPD-MtSEO-F4-HXK2, 424GPD-MtSEO-F1-PGI, 424GPD-MtSEO-F2-PGI, 424GPD-MtSEO-F4-PGI,) wurden jeweils in Hefen transformiert, die bereits ein Plasmid (425GPD-MtSEO-F1 oder 425GPD-MtSEO-F4) zur Herstellung artifizieller Forisome aus MtSEO-F1 oder MtSEO-F4 enthalten (siehe a)III.) Die resultierenden Doppelmutanten (z.B. 425GPD-MtSEO-F1/424GPD-MtSEO-F2-G6PDH) sind somit bzgl. der Leucin-, als auch bzgl. der Tryptophan-Auxotrophie komplementiert.
V. Expressionshefen, die enzymgekoppelte Forisome produzieren (siehe a)I.-IV.), wurden in einem Maßstab von 50 ml bis zu einer OD₆₀₀ₙₘ von 5-7 kultiviert und mittels Zentrifugation (1000 x g, 10 min) geerntet. Das Hefepellet wurde mit 50 ml V-Medium (10 mM TRIS, 10 mM EDTA, 100 mM KCl, pH 7.4) gewaschen und nach erneuter Zentrifugation (1000 x g, 10 min) bei -20°C eingefroren. Das gefrorene Zellpellet wurde in 1 ml V-Medium resuspendiert und mit ca. 500 mg Glaskugeln (425-600 µm) versetzt. Der Zellaufschluss erfolgte in 1.5 ml Gefäßen bei 30 Hz in der Schwingmühle MM400 (Retsch, Deutschland). Im Folgenden wurden die artifiziellen Forisome mit den unlöslichen Zellbestandteilen abzentrifugiert und in 0.5 ml V-Medium resuspendiert. Die Lösung wurde auf einen Sucrose- oder Nycodenz-Dichtegradienten gegeben, in dem die Sucrose- oder Nycodenz-Konzentration im Gradienten von 40% auf 70% steigt. Der Gradient wurde in einer Beckman-Ultrazentrifuge bei 163.000xg bei 4°C für 3 h zentrifugiert.
   Danach wurde die forisomenhaltige Phase im Gradienten mit einer Pipette aufgenommen, 1:2 mit V-Medium verdünnt und in 2 gleichgroße Aliquots aufgeteilt. Die Aliquots wurden für 10 Minuten bei 100 x g abzentrifugiert und der Überstand abgenommen. Die Forisome des ersten Aliquots wurden anschließend in 50 µl V-Medium aufgenommen und für die Massen- und Konzentrationsbestimmung der enzymgekoppelten, artifiziellen Forisome mittels SDS-Polyacrylamidgelelektrophorese (SDS-PAGE) verwendet. Das zweite Aliquot wurde in 50 µl Enzympuffer aufgenommen (für G6PDH-gekoppelte Forisome: 250 mM Glycylglycinpuffer, pH 7,4; HXK2-gekoppelte Forisome: 0,05 M Tris-HCl Puffer mit 13,3 mM MgCl₂, pH 8; PGIgekoppelte Forisome: 250 mM Glycylglycinpuffer, pH 7,4). Dieses Aliquot wurde für die Aktivitätsbestimmung der forisomengekoppelten Enzyme mittels spezifischer Enzym-Assays verwendet.
VI. Enzymgekoppelte, artifizielle Forisome (siehe a)IV.) wurden bzgl. ihrer Masse und Konzentration mittels SDS-PAGE untersucht. Dabei werden die verschiedenen Forisomenproteine, aus denen die enzymgekoppelten, artifiziellen Forisome aufgebaut sind (z.B. MtSEO-F1 und MtSEO-F2-Enzymfusionsprotein) getrennt. Die Anwesenheit der einzelnen Proteine wurde durch den Vergleich ihrer bioinformatisch vorhergesagten Masse (z.B. MtSEO-F2-G6PDH = 124,7 Kilodalton) mit der tatsächlichen Masse des Proteins im Gel (MtSEO-F2-G6PDH = ca. 130 kDa) nachgewiesen. Die Konzentrationsbestimmung der Proteine erfolgte anhand einer zusätzlich aufgetragenen Standardreihe definierter Proteinmengen und/oder mithilfe des Proteinmarkers *Precision Plus Protein Standards unstained* (Bio-Rad). Aus einer 50 ml Hefeexpressionskultur erhalten wir, je nach gewähltem Forisomenprotein und Enzymfusion, eine Gesamtproteinmenge (einzelnes MtSEO-F Protein + MtSEO-F-Enzymfusion) von 56-124 µg artifizielle, enzymgekoppelte Forisome. Der Anteil der MtSEO-F-Enzymfusion am Gesamtproteingehalt beträgt dabei, je nach Fusionspartner, 10% - 50%. Die größten Mengen, sowohl an Gesamtprotein (124 µg/ 50 ml Kultur) als auch an Enzymfusionsprotein (37 µg/ 50 ml Kultur) erzielten wir bei der Herstellung von PGI-gekoppelten Enzymforisomen (MtSEO-F1/MtSEO-F2-PGI).

Die Aktivität der forisomenimmobilisierten Enzyme wurde mittels spezifischer spektrophotometrischer Enzym-Assays bestimmt. Für die Glukose-6-Phosphat-Dehydrogenase wurde das empfohlene Protokoll von Sigma-Aldrich (Deutschland) verwendet. Der Assay beruht auf der durch G6PDH katalysierten Konversion von Glukose-6-Phosphat zu 6-Phosphogluconolacton. Bei dieser Reaktion wird Nicotinamid-Adenin-Dinukleotid-Phosphat (NADP⁺) zu NADPH reduziert:

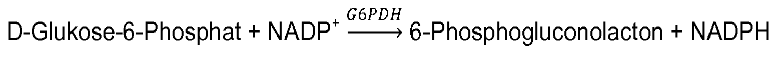

Die Extinktion von NADPH im Wellenbereich von 340 nm kann photometrisch gemessen werden und anhand dieser die Enzymaktivität berechnet werden. Für den Assay wurden die aufgereinigten Enzymforisome aus dem zweiten Aliquot (siehe a)V.) eingesetzt. Mithilfe der ermittelten Konzentration der enzymgekoppelten Forisome (siehe a)VI.) konnten die gemessenen Enzymaktivitäten pro Gramm artifizielle Forisome errechnet werden. Man erhielt je nach Konstrukt (siehe a)III.) Aktivitäten von 2000-2700 Units pro Gramm artifizielle Forisome für die forisomenimmobilisierte Glukose-6-Phosphat-Dehydrogenase. Im Vergleich dazu besitzt die von Sigma-Aldrich (Deutschland) kommerziell erhältliche, auf Agarosekügelchen immobilisierte Glukose-6-Phosphat-Dehydrogenase lediglich 1000-1750 Units pro Gramm Agarose. Bei erfindungsgemäßer Verwendung weist die forisomenimmobilisierte Glukose-6-Phosphat-Dehydrogenase somit eine deutlich höhere spezifische Enzymaktivität (Enzymaktivität bezogen auf die Menge an Trägermaterial) auf. **Figur 6** zeigt einen Enzymaktivitätstest der Glukose-6-Phosphat-Dehydrogenase, welche an erfindungsgemäße Forisomenkörper aus SEO-F1 und SEO-F2 gekoppelt ist.

Auch die Aktivität der forisomenimmobilisierten Hexokinase 2 und Phosphoglucoisomerase wurde anhand eines ähnlichen Assay-Prinzips bestimmt. Dabei wurden lediglich zwei hintereinander ablaufende Enzymreaktionen genutzt, um die Enzymaktivität anhand der Absorptionszunahme von NADPH bei 340 nm zu messen. Für die Hexokinase 2 wurde das von Worthington (Lakewood, NJ, USA) empfohlene Protokoll verwendet, dem folgende Reaktion zugrunde liegt:

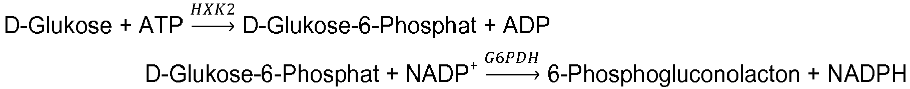

Die für die zweite Reaktion benötigte Glukose-6-Phosphatdehydrogenase wurde dem Assay-Ansatz in Form eines kommerziell erhältlichen, löslichen Enzyms mit definierter Aktivität zugefügt. Man erhielt je nach Konstrukt (siehe a)III.) Aktivitäten von 6000-8000 Units pro Gramm artifizieller Forisome für die forisomenimmobilisierte Hexokinase 2. Die von Sigma-Aldrich angebotene Agarose-immobilisierte Hexokinase weist dagegen lediglich eine Aktivität von 50-75 U auf.

Für die Phosphoglucoisomerase wurde das von Sigma-Aldrich (Deutschland) empfohlene Protokoll verwendet, dem folgende Reaktion zugrunde liegt:

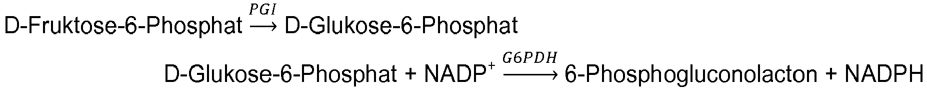

Man erhielt je nach Konstrukt (siehe a)III.) Aktivitäten von 6000-8000 Units pro Gramm artifizieller Forisome für die forisomenimmobilisierte Phosphoglucoisomerase. Die von Sigma-Aldrich angebotene Agarose-immobilisierte Phosphoglucoisomerase weist dagegen lediglich eine Aktivität von 300-600 U auf.

### Beispiel 2 - Aufreinigung von Proteinen

### 2.1a Aufreinigung rekombinanter Proteine mittels unmutierter Forisomengene oder in Abwesenheit unmutierter Forisomengene

I. Die codierende Sequenz eines Fragments des Malaria-Oberflächenantigens MSP (MSP1₁₉) wurde auf Basis eines Vektors mit den folgenden Oligonukleotiden amplifiziert (Schnittstellen sind unterstrichen).
   MSP1₁₉ NcoI fw: 5'- AGACCATGGACCTGCGTATTTCTCAG-3'
   MSP1₁₉ NcoI FaXa rev: 5'- AGACCATGGT*ACGACCTTCGATCC*TGCATATAGAAATGCC-3'
   MSP1₁₉ XhoI FaXa fw: 5'- AGACTCGAGAATCGAAGGTCGTGACCTGCGTATTTCTCAG-3'
   MSP1₁₉ XbaI rev: 5'- AGATCTAGATCACCTGCATATAGAAATG-3'

   Die Primer MSP1₁₉ NcoI FaXa rev und MSP1₁₉ XhoI FaXa fw enthalten neben den genspezifischen Sequenzen die codierende Sequenz der Erkennungsstelle für die Protease Factor Xa (in kursiv dargestellt). Das erste Amplifikat wurde mit dem Restriktionsenzym NcoI behandelt und in die NcoI-Schnittstelle der Vektoren pENTR4-MtSEO-F1 mit Stopcodon, pENTR4-MtSEO-F2 mit Stopcodon und pENTR4-MtSEO-F4 mit Stopcodon kloniert (siehe a)I.), um die Vektoren pENTR4-MSP1₁₉-MtSEO-F1, pENTR4-MSP1₁₉-MtSEO-F2 und pENTR4-MSP1₁₉-MtSEO-F4 zu erhalten. Das zweite Amplifikat wurde mit den Restriktionsenzymen XhoI und Xbal behandelt und in die XhoI/XbaI-Schnittstellen der Vektoren pENTR4-MtSEO-F1 ohne Stopcodon, pENTR4-MtSEO-F2 ohne Stopcodon und pENTR4-MtSEO-F4 ohne Stopcodon kloniert (siehe a)I.), um die Vektoren pENTR4-MtSEO-F1-MSP1₁₉, pENTR4-MtSEO-F2-MSP1₁₉ und pENTR4-MtSEO-F4-MSP1₁₉ zu erhalten. Zur Herstellung der Expressionsvektoren 424GPD-MSP1₁₉-MtSEO-F1, 424GPD-MSP1₁₉-MtSEO-F2, 424GPD-MSP1₁₉-MtSEO-F4, 424GPD-MtSEO-F1-MSP1₁₉, 424GPD-MtSEO-F2-MSP1₁₉ und 424GPD-MtSEO-F4-MSP1₁₉ wurden die hergestellten Vektoren mit dem Hefevektor 424GPD-ccdB (Addgene, USA) rekombiniert.
II. Die Vektoren 424GPD-MSP1₁₉-MtSEO-F4 und 424GPD-MtSEO-F4-MSP1₁₉ wurden in den Hefestamm InvSc1 (Invitrogen, Deutschland) transformiert. Zur Selektion wurde die Komplementierung der Tryptophan-Auxotrophie des Hefestammes genutzt. Die Fusionsproteine aus MSP1₁₉ und MtSEO-F4 bilden auch ohne zusätzliche Expression eines weiteren MtSEO-F-Proteins Forisomen aus.
   Die Vektoren 424GPD-MSP1₁₉-MtSEO-F1, 424GPD-MSP1₁₉-MtSEO-F2, 424GPD-MtSEO-F1-MSP1₁₉ und 424GPD-MtSEO-F2-MSP1₁₉ wurden in Hefen transformiert, die bereits ein Plasmid (425GPD-MtSEO-F1) zur Herstellung artifizieller Forisome aus MtSEO-F1 enthalten (siehe a)III.). Die resultierenden Hefen (z.B. 425GPD-MtSEO-F1/424GPD-MSP1₁₉-MtSEO-F1) sind bzgl. Ihrer Leucin- und Tryptophanauxotrophie komplementiert und stellen artifizielle Forisome in Fusion zu dem MSP1₁₉-Protein her.
III. Die artifiziellen Forisome in Fusion zu MSP1₁₉ wurden wie in 1.V. beschrieben aufgereinigt und mittels SDS-PAGE und Western Blot nachgewiesen und quantifiziert. Alle Konstrukte konnten zur Aufreinigung genutzt werden. Die beste Aufreinigung von 0,42 mg MSP1₁₉ pro Liter Zellkultur erzielten die Erfinder jedoch mit dem 424GPD-MSP1₁₉-MtSEO-F4-Konstrukt. Eine Optimierung durch die Anpassung von Kultivierung und Aufreinigung wird in Zukunft zur Aufreinigung von höheren Proteinmengen führen. Des Weiteren kann durch die Inkubation mit der Protease Factor Xa das MSP1₁₉-Protein vom artifiziellen Protein abgespalten werden. Außerdem haben die Erfinder beobachtet, dass bestimmte, reduzierende und calciumhaltige Pufferbedingungen (4 mM CaCl, 200 µM NaHSO₃, 10 mM TRIS, 100 mM KCl, pH 7.2) zur Auflösung artifizieller Forisome führen können (insbesondere bei Mutationen der Cysteine in Position 615 und 620 des MtSEO-F1-Proteins). Dieser Übergang von der unlöslichen Phase in die lösliche Phase kann auch zur Proteinaufreinigung genutzt werden. **Figur 7** zeigt die Aufreinigung von MSP1₁₉ mit Hilfe von Forisomenkörpern aus SEO-F1 oder SEO-F4. Dargestellt ist die immunologische Detektion von MSP1₁₉.

### 2.1b Aufreinigung rekombinanter Proteine mittels cystein-mutierter Forisomengene

Die Cysteine Nummer 3 und 8 im Sequenzlogo (Figur 5) wurden im MtSEO-F1 Gen mit dem QuikChange II Site-Directed Mutagenesis Kit von Agilent Technologies (CA, USA) nach Herstellerangaben zu Serinen mutiert. Als Basis diente der Vektor pENTR4-MtSEO-F1 mit und ohne Stopcodon (Bespiel 1). Die Cysteine Nummer 3 und Nummer 8 entsprechen der 615. und 620. Aminosäure des MtSEO-F1-Proteins. Das resultierende, mutierte MtSEO-F1-Gen wird im Folgenden deshalb MtSEO-F1 (C615S/C620S) genannt.

Die codierende Sequenz eines Fragments des Malaria-Oberflächenantigens MSP (MSP1₁₉) wurde wie unter Beispiel 2.1a beschrieben im Vektor pENTR4™-Vektors (Invitrogen, Deutschland) *up-* und *downstream* zu MtSEO-F1 (C615S/C620S) kloniert. Mittels Rekombination des Vektors pENTR4-MtSEO-F1(C615S/C620S) mit dem Hefevektoren 425GPD-ccdB (Addgene, USA) und Rekombination der Vektoren pENTR4-MSP1₁₉-MtSEO-F1 (C615S/C620S) und pENTR4-MtSEO-F1(C615S/C620S)-MSP1₁₉ mit dem Hefevektoren 424GPD-ccdB (Addgene, USA) wurden die Expressionsvektoren 425GPD-MtSEO-F1 (C615S/C620S), 424GPD-MSP1₁₉-MtSEO-F1(C615S/C620S), 424GPD-MtSEO-F1(C615S/C620S)-MSP1₁₉.

Die Hefevektoren wurden in den folgenden Kombinationen
425GPD-MtSEO-F1 (C615S/C620S) + 424GPD-MSP1₁₉-MtSEO-F1(C615S/C620S) und
425GPD- MtSEO-F1 (C615S/C620S) + 424GPD-MtSEO-F1(C615S/C620S)-MSP1₁₉
in den Hefestamm InvSc1 (Invitrogen, Deutschland) transformiert. Zur Selektion wurde die Komplementierung der Leucin- und Tryptophan-Auxotrophie des Hefestammes genutzt. Die resultierenden Hefen produzieren artifizielle Forisome aus MtSEO-F1(C615S/C620S), die MSP1₁₉-Protein enthalten.

Die resultierenden artifiziellen Forisome lassen sich in reduzierendem Puffer mit Calciumionen (4 mM CaCl, 200 µM NaHSO₃, 10 mM TRIS, 100 mM KCl, pH 7.2) zu fast 100% in die lösliche Form überführen, während in der nicht mutierten Version nur ein geringer Anteil in die lösliche Form übergeht.

Damit kann der Aufreinigungsprozess verkürzt werden. Nach Kultivierung können die Hefezellen, die artifizielle Forisome mit MSP1₁₉-Protein enthalten, aufgeschlossen werden, die artifiziellen Forisome und Hefetrümmer von löslichen Bestandteilen durch Zentrifugation getrennt werden und dann die Protein-Forisom-Fusionen in die lösliche Phase überführt werden.

### 2.2 Aufreinigung von Antikörpern mittels artifizieller Forisome

I. Die codierende Sequenz des Small rubber particle proteins 3 (SRRP3) wurde auf Basis eines Vektors mit den folgenden Oligonukleotiden amplifiziert (Restriktionsenzyme sind unterstrichen).
   SRPP3 XhoI fw: 5'-AGA CTCGAG A ATGACCGACGCTGCTTC-3'
   SRPP 3 XhoI rev: 5'-AGA CTCGAG TCATGTTTCCTCCACAATC-3'

   Das Amplifikat wurde mit dem Restriktionsenzym XhoI behandelt und in die XhoI-Schnittstelle des Vektors pENTR4-MtSEO-F1 ohne Stopcodon kloniert (siehe a)I.), um den Vektor pENTR4-MtSEO-F1-SRPP3 zu erhalten. Zur Herstellung des Expressionsvektors 424GPD-MtSEO-F1-SRPP3 wurde der hergestellte Vektor mit dem Hefevektor 424GPD-ccdB (Addgene, USA) rekombiniert.
II. Der Vektor 424GPD-MtSEO-F1-SRPP3 wurde in Hefen transformiert, die bereits ein Plasmid (425GPD-MtSEO-F1) zur Herstellung artifizieller Forisome aus MtSEO-F1 enthalten (siehe 1.III.). Die resultierenden Hefen (z.B. 425GPD-MtSEO-F1/424GPD- MtSEO-F1-SRPP3) sind bzgl. ihrer Leucin- und Tryptophanauxotrophie komplementiert und stellen artifizielle Forisome in Fusion zu dem SRPP3-Protein her. Die Hefen wurden im Maßstab von 50 ml bis zu einer OD₆₀₀ kultiviert, abzentrifugiert und in 1 ml V-Medium (10 mM TRIS, 10 mM EDTA, 100 mM KCl, pH 7.4) mittels einer Kugelmühle aufgeschlossen. Die artifiziellen Forisome, die das Antigen tragen, lagen nun frei in der Lösung vor und konnten im Folgenden für die Aufreinigung polyklonaler oder monoklaner Antikörper genutzt werden.
III. Die artifiziellen Forisome mit Antigen wurden mit 500 µl eines polyklonalen Antikörperserums gegen SRPP3, der in einem Hasen produziert wurde, für 30 Minuten inkubiert. Dabei banden die spezifischen Antikörper an die artifiziellen Forisome. Die Forisome wurden abzentrifugiert (4000 x g, 4 min) und dreimal mit 1 ml PBS (137 mM NaCl, 2.7 mM KCl, 10 mM Na₂HPO₄, 2 mM KH₂PO₄, pH 7.4) gewaschen. Dann wurden die Antikörper mit 450 µl 0.1 M Glycine-HCl-Lösung (pH 2.7) für 5 min eluiert. Daraufhin wurde die Antikörperlösung mit 50 µl 1M Tris-HCl-Lösung (pH 8.5) neutralisiert. In nachfolgenden Blots konnte die hohe Spezifität des aufgereinigten Antikörpers (ohne Serumverunreinigung) nachgewiesen werden. Die mittels der Forisomentechnologie aufgereinigten Antikörper konnten für verschiedene Anwendungen genutzt werden (Western Blot, Immunopräzipitation ELISA, Antikörpertherapie, etc.). Das Prinzip dieser Aufreinigung ist in **Figur 7** dargestellt. **Figur 8** zeigt die Nutzung der Interaktion von B-Domäne mit IgG-Antikörper zur Immobilisierung von artifiziellen Forisomen. Ein artifizielles Forisom, das aus mit der B-Domäne gekoppelten SEO-F1-Untereinheiten besteht, bindet fluoreszierende IgG-Antikörper.

### Beispiel 3 - Befestigung artifizieller Forisome an technische Oberflächen (Oberflächenkopplung)

I. Die codierende Sequenz der Glutathion-S-Transferase (GST) wurde auf Basis des pGex-3X-Vektors (GE Healthcare, USA) mit folgenden Oligonukleotiden amplifiziert (Restriktionsschnittstellen sind unterstrichen).
   GST fw NcoI XhoI fw: 5'-AGA CCA TGG GAC TCG AGA ATG TCC CCT ATA CTA GGT TA-3'
   GST SalI rev: 5'-AGA GTC GAC TTA ACG ACC TTC GAT CAG ATC-3'

   Das Fragment wurde mit den Restriktionsenzymen NcoI/SalI behandelt und in den NcoI/XhoIgeschnittenen pENTR4™-Vektor kloniert, was in dem Vektor pENTR4-GST resultierte. Im Folgenden wurde das Amplifikat des MtSEO-F1-Gens mit Stop (siehe 1.I.) in die Ncol/Xhol-Stellen des resultierenden Vektors kloniert, um den Vektor pENTR4-GST-MtSEO-F1 zu erhalten. Zur Herstellung des Expressionsvektors 424GPD-GST-MtSEO-F1 wurde der Vektor pENTR4-GST-MtSEO-F1 mit dem Hefevektor 424GPD-ccdB (Addgene, USA) rekombiniert. Der Expressionsvektor wurde in Hefen transformiert, die bereits ein Plasmid (425GPD-MtSEO-F1) zur Herstellung artifizieller Forisome aus MtSEO-F1 enthalten (siehe a)III.). Die resultierenden Hefen (425GPD-MtSEO-F1/424GPD-GST-MtSEO-F1) sind bzgl. ihrer Leucin- und Tryptophanauxotrophie komplementiert und stellen artifizielle Forisome mit einem GST-Tag her. Sie wurden, wie in a)V. beschrieben, aufgereinigt und mittels SDS-PAGE konnte das Vorhandensein der jeweiligen Proteine (MtSEO-F1 und GST-MtSEO-F1) nachgewiesen werden. Des Weiteren konnte gezeigt werden, dass die resultierenden, artifiziellen GSTgekoppelten Forisome an einer Glutathion-gekoppelten Matrix (Glutathione Sepharose 4B, Amersham Bioscience, USA) adhärieren.
II. Die codierende Sequenz der B-Domäne aus dem Protein A von *Staphylococcus aureus* wurde auf Basis des Vektors 424GPD-ccdB-TAP (Addgene, USA) mit den folgenden Oligonukleotiden amplifiziert (Restriktionsschnittstellen sind unterstrichen).
   B-Domäne NcoI fw: 5'- AGACCATGGCGGATAACAAATTCAACA-3'
   B- Domäne NcoI rev: 5'-AGACCATGGCTTTTGGTGCTTGAGCATC-3'
   B- Domäne XhoI fw: 5'- AGACTCGAGAGCGGATAACAAATTCAAC-3'
   B- Domäne XhoI rev: 5'- AGACTCGAGTCATTTTGGTGCTTGAGCATC-3'

Das erste Amplifikat wurde mit dem Restriktionsenzym NcoI behandelt und in die NcoI-Schnittstelle der Vektoren pENTR4-MtSEO-F1 mit Stopcodon und pENTR4-MtSEO-F4 mit Stopcodon kloniert (siehe a)I.), um die Vektoren pENTR4-B-Domäne-MtSEO-F1 und pENTR4-B-Domäne-MtSEO-F4 zu erhalten. Das zweite Amplifikat wurde mit dem Restriktionsenzym Xhol behandelt und in die XhoI-Schnittstelle der Vektoren pENTR4-MtSEO-F1 ohne Stopcodon und pENTR4-MtSEO-F4 ohne Stopcodon kloniert (siehe a)I.), um die Vektoren pENTR4-MtSEO-F1-B-Domäne und pENTR4-MtSEO-F4-B-Domäne zu erhalten. Zur Herstellung der Expressionsvektoren 424GPD-B-Domäne-MtSEO-F1, 424GPD-B-Domäne-MtSEO-F4, 424GPD-MtSEO-F1-B-Domäne, 424GPD-MtSEO-F4-B-Domäne wurden die hergestellten Vektoren mit dem Hefevektor 424GPD-ccdB (Addgene, USA) rekombiniert.

Die Vektoren 424GPD-B-Domäne-MtSEO-F4 und 424GPD-MtSEO-F4-B-Domäne wurden in den Hefestamm InvSc1 (Invitrogen, Deutschland) transformiert. Zur Selektion wurde die Komplementierung der Tryptophan-Auxotrophie des Hefestammes genutzt. Die Fusionsproteine aus B-Domäne und MtSEO-F4 bilden ohne zusätzliche Expression eines weiteren MtSEO-F-Proteins forisomenähnliche Strukturen aus.

Die Vektoren 424GPD-B-Domäne-MtSEO-F1 und 424GPD-MtSEO-F1-B-Domäne wurden in Hefen transformiert, die bereits ein Plasmid (425GPD-MtSEO-F1) zur Herstellung artifizieller Forisome aus MtSEO-F1 enthalten (siehe 1.III.). Die resultierenden Hefen (z.B. 425GPD-MtSEO-F1/424GPD-B-Domäne-MtSEO-F1) sind bzgl. ihrer Leucin- und Tryptophanauxotrophie komplementiert und stellen artifizielle Forisome in Fusion zu einer B-Domäne her. Alle hergestellten, artifiziellen Forisome mit B-Domäne immobilisieren an IgG-gekoppelter Sepharose (GE Healthcare, USA).

### 2.3 Herstellung und Aufreinigung von artifiziellen SEO-F-Forisomen aus zwei unterschiedlichen SEO-F-Proteinen

### 2.3.1: Eine Fusion aus Aminosäure 1-96 von MtSEO-F4 und aus der Aminosäure 73-648 von MtSEO-F1.

Das N-terminale MtSEO-F4-Fragment und das C-terminale MtSEO-F1-Fragmen wurden mit den Oligonukleotiden
MtSEO-F4 MSLSN NcoI fw: 5'-AGACCATGGGATCCCTTTCCAACTTAGGAAGTG -3'
MtSEO-F4 LISCQ NcoI rev: 5'-AGACCATGGCCTGACAAGAAATCAGCTT-3'
MtSEO-F1 MITTR NcoI fw: 5'-AGACCATGGGAATGATAACCACCCCTC-3'
MtSEO-F1 QNGKI XhoI rev: 5'-AGACTCGAGGTCATATCTTGCCATTCTGTGGAG-3'
amplifiziert und in den NcoI/XhoI-restringierten pENTR4 Vektor kloniert. Danach wurde der entstandene Vektor pENTR4-MtSEO-F4(1-288bp)/MtSEO-F1 (219-1944 bp) mit dem Pflanzenexpressionsvektor pBatTL und dem Hefeexpressionsvektor 425GPD-ccdB rekombiniert. Der entstandene pBatTL-MtSEO-F4(1-288bp)/MtSEO-F1(219-1944bp) wurde in Agrobakterien transformiert, mit denen *N. benthamiana-Pflanzen* infiltriert wurden (siehe Müller *et al.,* 2010). Der entstandene Vektor 425GPD-MtSEO-F4(1-288 bp)/MtSEO-F1(219-1944 bp) wurde in den Hefestamm InvSc1 transformiert. In beiden Systemen konnte die Ausbildung artifizieller Forisome mikroskopisch beobachtet werden. Die Aufreinigung wurde, wie zuvor unter Beispiel 1 V beschrieben, durchgeführt.

### 2.3.2: Eine Fusion aus den Aminosäuren 1-583 von MtSEO-F1 und aus den Aminosäuren 620-670 von MtSEO-F2.

Das N-terminale MtSEO-F1-Fragment und das C-terminale MtSEO-F2-Fragment wurden mit den Oligonukleotiden
MtSEO-F1 MSLNS NcoI fw: 5'-AGACCATGGGATCATTGTCCAATGGAACTA-3'
MtSEO-F1 FKEYY XhoI rev: 5'-AGACTCGAGTGATAGTATTCTTTGAATGCAAT-3'
MtSEO-F2 DTKLS XhoI fw: 5'-AGACTCGAGTGATACTAAGCTTTCAGAGAT-3'
MtSEO-F2 DSCCI XhoI bw: 5'-AAACTCGAGTCAAATGCAGCAACTATCTGGATCATC-3'
amplifiziert und in den NcoI/XhoI-restringierten pENTR4 Vektor kloniert. Danach wurde der entstandene Vektor pENTR4-MtSEO-F1(1-1749 bp)/MtSEO-F2(1860-2010 bp) mit dem Pflanzenexpressionsvektor pBatTL und dem Hefeexpressionsvektor 425GPD-ccdB rekombiniert. Der entstandene pBatTL-MtSEO-F1(1-1749 bp)/MtSEO-F2(1860-2010 bp) wurde in Agrobakterien transformiert, mit denen *N. benthamiana-Pflanzen* infiltriert wurden (siehe Müller *et al.,* 2010). Der entstandene Vektor 425GPD-MtSEO-F1(1-1749 bp)/MtSEO-F2(1860-2010 bp) wurde in den Hefestamm InvSc1 transformiert. In beiden Systemen konnte die Ausbildung artifizieller Forisome mikroskopisch beobachtet werden. Die Aufreinigung wurde, wie zuvor unter Beispiel 1 V beschrieben, durchgeführt.

## Patentansprüche

1. Verwendung eines artifiziellen Forisomenkörpers zur Enzymimmobilisierung oder zur Aufreinigung rekombinanter Proteine, wobei der artifizielle Forisomenkörper ein Fusionsprotein aus mindestens einem SEO-F-Protein oder einem mindestens 50 Aminosäuren umfassenden Teilstück davon und mindestens einem weiteren Protein oder Peptid umfasst, mit Ausnahme von Forisomenkörpern, die ausschließlich oder teilweise aus einem fluoreszierenden Fusionsprotein aufgebaut sind, wobei
(a) das weitere Protein oder Peptid eine Masse von maximal 30 kDa aufweist und der Forisomenkörper kein unfusioniertes SEO-F-Protein oder eine C-terminal um bis zu 45 Aminosäuren und/oder N-terminal um bis zu 13 Aminosäuren deletierte Form dieses Proteins umfasst, oder
(b) der Forisomenkörper weiterhin ein unfusioniertes SEO-F-Protein oder eine C-terminal um bis zu 45 Aminosäuren und/oder N-terminal um bis zu 13 Aminosäuren deletierte Form dieses Proteins umfasst, wobei das unfusionierte SEO-F-Protein aus solchen Proteinen ausgewählt ist, die die Eigenschaft besitzen, in Abwesenheit weiterer SEO-F-Proteine homomere Forisomenkörper ausbilden zu können, oder
(c) das weitere Protein oder Peptid ein Teilstück eines zweiten, vom ersten SEO-F-Protein verschiedenen SEO-F-Proteins ist, mit der Maßgabe, dass eines der beiden SEO-F-Proteine in unfusionierter Form in der Lage ist, homomere Forisomenkörper auszubilden, und das Fusionsprotein aus einem N-terminalen SEO-F-Protein-Teilstück sowie einem C-terminalen SEO-F-Protein-Teilstück besteht, wobei die Teilstücke ein SEO-F-Protein darstellen, das vollständig ist oder das C-terminal um bis zu 50 Aminosäuren und/oder N-terminal um bis zu 13 Aminosäuren deletiert ist,
mit der Maßgabe, dass ggf. eine beliebige Anzahl der vier Cysteine, die in den nativen SEO-F-Proteinen im C-terminalen Abschnitt zwischen AS 600 und AS 670 vorhanden sind, gegen Aminosäuren ausgetauscht sein können, die keine S-S-Brückenbindung ausbilden können.

2. Verwendung nach Anspruch 1, wobei Variante (c), ein Teilstück eines SEO-F1-Proteins, vorzugsweise des MtSEO-F1-Proteins, enthält.

3. Verwendung nach Anspruch 1, worin das weitere Protein oder Peptid ausgewählt ist unter Proteinen, die dem Stoffumsatz dienen, Proteinen, die eine Immunreaktion auslösen können und/oder einen therapeutischen Nutzen haben, und Proteinen, die für biotechnologische Anwendungen nutzbar sind, insbesondere unter Enzymen, Antikörpern, Antigenen sowie solchen Proteinen oder Peptiden, die sich aufgrund einer Affinitätsreaktion mit einem substratgebundenen biologischen oder biochemisch erzeugten Material auf einem Substrat immobilisieren lassen.

4. Verwendung nach Anspruch 3, worin das Fusionsprotein ein Enzym aufweist, das an das N-terminale Ende des SEO-F-Proteins oder des Teilstücks davon fusioniert ist, oder worin das Fusionsprotein ein Protein aufweist, das einen therapeutischen Nutzen hat oder für biotechnologische Anwendungen nutzbar ist und das an das C-terminale Ende des SEO-F-Proteins oder des Teilstücks davon fusioniert ist.

5. Verwendung nach einem der Ansprüche 3 und 4, umfassend mindestens zwei Fusionsproteine, die jeweils ein Enzym aufweisen, derart, dass das Produkt der Umsetzung eines Substrats mit einem ersten dieser Enzyme als Substrat für ein zweites dieser Enzyme dienen kann.

6. Verwendung nach einem der voranstehenden Ansprüche, worin das SEO-F-Protein oder das mindestens 50 Aminosäuren aufweisende Teilstück davon ein Protein ist, ausgewählt unter einem Protein mit einer der Sequenzen SEQ ID-No: 1 bis 8 oder einem mindestens 50 Aminosäure aufweisenden Teilstück davon.

7. Verwendung nach einem der voranstehenden Ansprüche, worin das unfusionierte SEO-F-Protein oder die C-terminal um bis zu 45 Aminosäuren und/oder N-terminal um bis zu 13 Aminosäuren deletierte Form dieses Proteins wie in Variante (b) des Anspruchs 1 definiert ausgewählt ist unter einem Protein mit einer der Sequenzen SEQ ID-No: 1, 4, 5, 6, 7 und 8.

## Claims

1. A use of an artificial forisome body for enzyme immobilisation or for purification of recombinant proteins, wherein the artificial forisome body comprises a fusion protein of at least one SEO-F-protein or an at least 50-amino acid comprising portion thereof, and at least one further protein or peptide, with the exception of forisome bodies that are composed exclusively or partly of a fluorescent fusion protein, wherein
(a) the further protein or peptide has a mass of at most 30 kDa, and the forisome body does not comprise an unfused SEO-F-protein or a form of said protein having C-terminal deletions of up to 45 amino acids and/or N-terminal deletions of up to 13 amino acids, or
b) the forisome body moreover comprises an unfused SEO-F-protein or a form of said protein having C-terminal deletions of up to 45 amino acids and/or N-terminal deletions of up to 13 amino acids, wherein the unfused SEO-F-protein is selected from proteins having the property of being capable of forming homomeric forisome bodies in the absence of further SEO-F-proteins, or
(c) the further protein or peptide is a portion of a second SEO-F-protein being different from the first SEO-F-protein, with the proviso that one of the two SEO-F-proteins in its unfused form is capable of forming homomeric forisome bodies, and the fusion protein is comprised of an N-terminal SEO-F-protein-portion and a C-terminal SEO-F-protein-portion, wherein the portions represent a SEO-F-protein that is complete or C-terminally deleted by up to 50 amino acids and/or N-terminally deleted by up to 13 amino acids,
with the proviso that optionally any number of the four cysteines located in the C-terminal portion between AS 600 and AS 670 of the native SEO-F-proteins, may be replaced by amino acids that are not capable of forming a S-S-bond.

2. The use according to claim 1, wherein variant (c) contains a portion of a SEO-F1-protein, preferably the MtSEO-F1-protein.

3. The use according to claim 1, wherein the further protein or peptide is selected from proteins that contribute to metabolism, proteins capable of triggering an immune response and/or having a therapeutic benefit, and proteins that are useful for biotechnological applications, in particular among enzymes, antibodies, antigens as well as those proteins or peptides that can be immobilized on a substrate due to an affinity reaction with a substrate-bound biological or biochemically produced material.

4. The use according to claim 3, wherein the fusion protein comprises an enzyme fused to the N-terminal end of the SEO-F-protein or the portion thereof, or wherein the fusion protein comprises a protein that has a therapeutic benefit or is useful for biotechnological applications, and which is fused to the C-terminal end of the SEO-F-protein or the portion thereof.

5. The use according to one of claims 3 and 4, comprising at least two fusion proteins, each comprising an enzyme, such that the product of the reaction of one substrate with a first of said enzymes can serve as a substrate for a second of said enzymes.

6. The use according to one of the preceding claims, wherein the SEO-F-protein or the portion thereof comprising at least 50 amino acids is a protein selected from a protein having one of the sequences of SEQ ID-No: 1 to 8 or a portion thereof comprising at least 50 amino acids.

7. The use according to one of the preceding claims, wherein the unfused SEO-F protein or the form of said protein having C-terminal deletions of up to 45 amino acids and/or N-terminal deletions of up to 13 amino acids as defined in variant (b) of claim 1 is selected from a protein having one of the sequences of SEQ ID-No: 1, 4, 5, 6, 7 and 8.

## Revendications

1. Utilisation d'un corps de forisome artificiel pour l'immobilisation d'enzymes ou pour la purification de protéines recombinantes, le corps de forisome artificiel comportant une protéine de fusion en au moins une protéine SEO-F ou un fragment de celle-ci comportant au moins 50 acides aminés et au moins une autre protéine ou un peptide, à l'exception des corps de forisomes constitués exclusivement ou partiellement d'une protéine de fusion fluorescente,
(a) l'autre protéine ou peptide comportant une masse de 30 kDa au maximum et le corps de forisome ne comportant pas une protéine SEO-F non fusionnée ou une forme C-terminale délétée à raison de jusqu'à 45 acides aminés, et/ou N-terminale délétée à raison de jusqu'à 13 acides aminés de cette protéine, ou
(b) le corps de forisome comportant en outre une protéine SEO-F non fusionnée ou une forme C-terminale délétée à raison de jusqu'à 45 acides aminés, et/ou N-terminale délétée à raison de jusqu'à 13 acides aminés de cette protéine, la protéine SEO-F non fusionnée étant choisie parmi des protéines telles qu'elles possèdent la propriété de pouvoir former en absence d'autres protéines SEO-F des corps de forisomes homomères, ou
(c) l'autre protéine ou peptide étant un fragment d'une deuxième protéine SEO-F différente de la première protéine SEO-F, avec la condition qu'une des deux protéines SEO-F, sous forme non fusionnée, est en mesure de former des corps de forisomes homomères et que la protéine de fusion consistant en un fragment N-terminal de protéine SEO-F ainsi qu'en un fragment C-terminal de protéine SEO-F, les fragments représentant une protéine SEO-F qui est complète, ou le fragment C-terminal étant délété à raison de jusqu'à 50 acides aminés, et/ou le fragment N-terminal étant délété à raison de jusqu'à 13 acides aminés,
avec la condition que, le cas échéant, un nombre quelconque des quatre cystéines qui sont présentes dans les protéines SEO-F natives dans le segment C-terminal entre AS 600 et AS 670 peuvent être remplacées par des acides aminés qui ne peuvent pas former de ponts S-S.

2. Utilisation selon la revendication 1, la variante (c) contenant un fragment d'une protéine SEO-F1, de préférence d'une protéine MtSEO-F1.

3. Utilisation selon la revendication 1, l'autre protéine ou peptide étant choisie parmi des protéines servant au taux métabolique, des protéines pouvant déclencher une réaction immunitaire et/ou ayant un bénéfice thérapeutique et des protéines utilisables pour des applications biotechnologiques, surtout parmi des enzymes, des anticorps, des antigènes ainsi que des protéines ou peptides susceptibles, par suite d'une réaction d'affinité avec un matériau biologique ou produit biochimiquement, lié à un substrat, d'être immobilisés sur ledit substrat.

4. Utilisation selon la revendication 3, la protéine de fusion comportant un enzyme fusionnée à l'extrémité N-terminale de la protéine SEO-F1 ou au fragment de celle-ci, ou la protéine de fusion comportant une protéine ayant un bénéfice thérapeutique ou étant utilisable pour des applications biotechnologiques et qui est fusionnée à l'extrémité C-terminale de la protéine SEO-F1 ou au fragment de celle-ci.

5. Utilisation selon l'une quelconque des revendications 3 et 4, comportant au moins deux protéines de fusion, chacune comportant une enzyme de telle manière que le produit de la réaction d'un substrat avec une première desdites enzymes peut servir de substrat pour une deuxième desdites enzymes.

6. Utilisation selon l'une quelconque des revendications précédentes, la protéine SEO-F1 ou le fragment de celle-ci comportant au moins 50 acides aminés étant une protéine choisie parmi une protéine avec une des séquences SEQ-ID N°: 1 à 8 ou un fragment de celle-ci comportant au moins 50 acides aminés.

7. Utilisation selon l'une quelconque des revendications précédentes, la protéine SEO-F1 non fusionnée ou la forme C-terminale délétée à raison de jusqu'à 45 acides aminés, et/ou N-terminale délétée à raison de jusqu'à 13 acides aminés de cette protéine comme défini dans la variante (b) de la revendication 1 étant choisie parmi une protéine avec une des séquences SEQ-ID N°: 1,4, 5, 6, 7 et 8.
